# EUROPEAN PATENT SPECIFICATION

(11) **EP 2 413 977 B1**
(45) Date of publication and mention of the grant of the patent: **23.12.2015**
(21) Application number: 10757944.3
(22) Date of filing: 01.04.2010
(51) Int. Cl.: A61L 2/04, A61L 12/04, F26B 3/02, F26B 19/00, H05B 3/06

(54) **A DRYING ARRANGEMENT**
TROCKNERANORDNUNG
AGENCEMENT DE SÉCHAGE

(30) Priority: 03.04.2009 AU 2009901449
(43) Date of publication of application: 08.02.2012
(73) Proprietor: Qirx Pty Ltd, Mitchell, ACT 2911 (AU)
(72) Inventor: HADFIELD, Matthew, David, Dunlop ACT 2615 (AU); COURTNEY, Thomas, Rivett ACT 2911 (AU); STEVENS, Blake, Ngunnawal ACT 2913 (AU); STEVENS, Denis, Palmerston ACT 2913 (AU); BARRY, Vaughan, Dunlop ACT 2615 (AU)
(74) Representative: Piotrowicz, Pawel Jan Andrzej
(86) International application number: PCT/AU2010/000374
(87) International publication number: WO 2010/111744

(56) References cited:
- WO-A1-2007/129892
- WO-A1-2008/141365
- WO-A1-2008/141365
- WO-A2-03/074093
- AU-S- 321 397
- AU-S- 322 923
- AU-S- 323 912
- JP-A- 4 073 063
- US-A- 4 044 226
- US-A- 4 044 226
- US-A- 4 228 136
- US-A- 4 329 568
- US-A- 4 341 948
- US-A- 4 369 355
- US-A- 4 529 868
- US-B1- 6 870 137

## Description

### Field of the Invention

The present invention relates generally to contact lenses, and in particular, to an arrangement for reducing discomfort and possible infections associated with using contact lenses.

### Background

Contact eye lenses (hereinafter referred to simply as lenses) are becoming increasingly widespread. Unlike conventional spectacles, lenses are placed directly upon the surface of the wearer's eyes, which are both delicate and sensitive. The terms "placement upon the surface of the eyes" and "insertion into the eyes" are used interchangeably in this description.

Contact lenses are perceived by wearers to be more uncomfortable to wear than conventional spectacles. Contact lenses are also perceived to be more complex to clean and store than conventional spectacles, and to be subject to problems such as eye discomfort and possible eye infection.

Microbial growth in contact lens storage containers, which generally occurs in the presence of moisture in the containers, can constitute a significant hazard to contact lens wearers (potentially causing eye infection), and a strong disincentive to spectacle wearers who are using or considering the use of contact lenses.

Some current contact lens storage containers are infused with silver ions to reduce contamination on the storage container surface. The silver ions are gradually released as the case is exposed to moisture, and this maintains the antimicrobial agent at the surface of the case. However, it is believed that this approach may not always sufficiently prevent contamination.

Drying the contact lens storage container and the lids, which in one current arrangement is done by placing them on a surface exposed to the ambient temperature and humidity conditions and the natural air currents within a room, can lead to drying times exceeding 24 hours when the ambient temperatures are low or the ambient humidity is high. This is awkward and can be inconvenient for the wearer, and allows an extended time for microbes and mould growth. This timeframe also typically exceeds the usual time between uses of the storage case to store the lenses, and therefore the lens case may continually provide an environment suitable for microbial growth. Storage containers, left open within a room, may also become contaminated by ingress of airborne contaminants such as dust, microbes and mould spores

Drying the container thoroughly with a cloth or paper towel, according to another current arrangement can, if done properly, quickly produce a dry container. However, this is an awkward procedure, requires availability storage and use of sufficiently clean wiping material, can be inconvenient for the wearer, and may also transfer potentially contaminating material from the drying material to the container.

WO 2008/141365 A1 describes a contact lens warming apparatus adapted to warm a contact lens stored in a removably inserted lens container. The apparatus comprises a controller, a heatable conformal cavity and a heating element. The controller is adapted to warm the contact lens in the lens container in order to reduce discomfort otherwise felt by a wearer of the contact lens when inserting the unwarmed contact lens into their eye.

### Summary

The inventors have realized that (a) wearers perceptions in regard to the complexity of contact lens use and the problems of eye discomfort and possible eye infection and (b) the actual problems of eye discomfort and possible eye infection result from wearers reluctance to undertake complex and time consuming tasks associated with the wearing and maintenance of contact lenses.

The inventors have concluded that these problems can be overcome or at least ameliorated by providing simple technical solutions which, in a first arrangement, comprises a combined contact lens warming and drying device referred to as a tailored lens warming and drying arrangement (TLWDA). In this arrangement the contact lens warming function addresses the discomfort arising from inserting contact lenses at temperatures below body temperature into the eye by pre-warming the lenses making them more comfortable to insert in the eye. The drying function actively dries the contact lens storage container by heating the container surfaces to a temperature that significantly reduce the drying time of the container and lids when compared to containers being passively dried at ambient temperature and humidity and avoids the complexity and possible contamination arising from manual drying. Suitable orientation and/or shielding of the containers also avoids contamination arising from airborne particles, dust and mould spores. In one arrangement the TLWDA is implemented by integrating drying functionality in a user friendly convenient manner into a tailored lens warming arrangements (TLWA) to thus form a tailored lens warming and drying arrangement (TLWDA). In another arrangement the drying functionality is provided without provision of the warming functionality in a "bare" dryer configuration.

It is an object of the present invention to overcome or at least ameliorate some of the problems sometimes experienced or perceived by users of contact lenses.

Disclosed are arrangements, referred to as tailored lens warming and drying arrangements (or TLWDA's) that actively dry the lens storage container and associated lids. Additionally, TLWDA's may gently warm the contact lens prior to insertion into the wearer's eye, to thus reduce the discomfort otherwise often experienced by the wearer when inserting an unwarmed lens into their eye.

According to a first aspect of the present invention, there is provided a contact lens drying apparatus configured to dry a lens container being removably engaged with the apparatus in a drying configuration, said lens container comprising a container vessel and a lid, the lid being dried separately from the container vessel, the apparatus comprising: a controller; drying engagement means for removably engaging the container vessel and the lid, said drying engagement means comprising: a lower plate for engaging the container vessel, said lower plate being configured to receive heat from a heating element; and an upper plate for engaging the lid, said upper plate being joined to the lower plate in order to (i) heat the lid and (ii) dispose the lid in an orientation that shields the container vessel to reduce ingress of airborne contaminants; and the heating element; wherein the controller is configured to direct the heating element to dry the engaged container vessel and the lid thereby significantly reducing microbial activity in the container.

According to another aspect of the invention there is provided a heating module configured to mate with a drying engagement means housed in a housing module, the drying engagement means comprising a lower plate for engaging a contact lens container vessel, said lower plate being configured to receive heat from a heating element; and an upper plate for engaging a contact lens container lid, said upper plate being joined to the lower plate in order to (i) heat the lid and (ii) dispose the lid in an orientation that shields the container vessel to reduce ingress of airborne contaminants, said heating module comprising: a controller; and said heating element; wherein the controller is configured to direct the heating element, when the heating module is mated with the drying engagement means, to dry the container vessel and said lid that are removably engaged with the drying engagement means according to a timing and temperature profile that is adapted for drying, the lid being dried separately from the container vessel.

According to yet another aspect of the invention there is provided a drying engagement means housed in a housing module, wherein the drying engagement means is configured to mate with a heating module which, when mated with the drying engagement means, is configured to dry a lens container comprising a container vessel and a lid that is removably engaged with the drying engagement means, the lid being dried separately from the container vessel, said drying engagement means comprising: a lower plate for engaging the container vessel, said lower plate being configured to receive heat from the engaged heating module; an upper plate for engaging the lid, said upper plate being joined to the lower plate in order to (i) heat the lid and (ii) dispose the lid in an orientation that shields the container vessel to reduce ingress of airborne contaminants; and means for mating with the heating module.

Other aspects of the invention are also disclosed.

### Brief Description of the Drawings

One or more embodiments of the present invention will now be described with reference to the drawings and appendices, in which:
**Fig. 1** shows a mechanical representation of one example of a single-component TLWA;
**Fig. 2** shows an electrical (control) representation of the TLWA of **Fig. 1****;**
**Fig. 3** shows a process of how the TLWA of **Fig. 1** can be operated;
**Fig. 4** shows a mechanical representation of another example of a single-component TLWA;
**Figs. 5(a) - 5(d)** show one example of a schematic diagram for the electrical (control and heating) aspects of the TLWA of **Fig. 1****;**
**Fig. 6** shows one example of a flow chart depicting a process used by the TLWA of **Fig. 1****;**
**Fig. 7** shows a mechanical representation of a first example of a two-component TLWA;
**Fig. 8** shows a mechanical representation of a second example of a two-component TLWA;
**Fig. 9** shows a mechanical exploded representation of a third example of a two-component TLWA together with lens container lids and vessels;
**Fig. 10** shows the two-component TLWA of **Fig. 9** in assembled form without the lens containers;
**Fig. 11** shows the two-component TLWA of **Fig. 9** together with the lens containers;
**Fig. 12** shows a mechanical representation of a first example of a two-component TLWDA, with lens containers mounted for warming;
**Fig. 13** shows the two-component TLWDA of **Fig. 12** with lens container lids and vessels oriented for drying;
**Fig. 14** shows an exploded mechanical representation of a second example of a two- component TLWDA with lens container vessels mounted for warming or drying;
**Fig. 15** shows the TLWDA of **Fig. 14** in assembled configuration without the lens containers;
**Fig. 16** shows the TLWDA of **Fig. 14** with lens container components oriented for warming or drying;
**Fig. 17** shows an exploded mechanical representation of a third example of a two- component TLWDA, which does not fall within the scope of the claims *per se,* with lens container vessels and lids oriented for drying;
**Fig. 18** shows the TLWDA of **Fig. 17** with lens container vessels and lids mounted for drying;
**Fig. 19** shows the TLWDA of **Fig. 17** without lens containers;
**Fig. 20** shows the TLWDA of **Fig. 12** without lens container;
**Fig. 21** shows a process of how the TLWDAs of **Figs. 12****,** **14** and **17** can be operated;
**Fig. 22** shows the two-component TLWDA of **Fig. 12** with lens container lids and vessels oriented for warming;
**APPENDIX A** set out a pseudo-code implementation of the flow chart of **Fig. 6****;** and
**APPENDIX B** set out a pseudo-code implementation of the flow chart of **Fig. 21****.**

### Detailed Description including Best Mode

Where reference is made in any one or more of the accompanying drawings to features which have the same reference numerals, those features have for the purposes of this description the same function(s) or operation(s), unless the contrary intention appears.

It is to be noted that the discussions contained in the "Background" section and that above relating to prior art arrangements relate to discussions of arrangements which form public knowledge through their use. Such discussions should not be interpreted as a representation by the present inventor(s) or the patent applicant that such arrangements in any way form part of the common general knowledge in the art.

As previously noted, in one arrangement the TLWDA is implemented by integrating drying functionality in a user friendly convenient manner into a tailored lens warming arrangements (TLWA) to thus form tailored lens warming and drying arrangements (TLWDA). The TLWA is now described after which the TLWDA is described.

### Tailored Lens Warming Arrangements (TLWA)

For both feelings of general wellbeing, and for reasons of safety, it is desirable to minimise any discomfort experienced by the wearer of the lenses, particularly when inserting the lenses into the eyes, in order to avoid the wearer flinching, and possibly injuring themselves as a result.

University studies commissioned by the Applicant have been carried out and confirm that there is a significant improvement in user comfort when contact lenses are warmed to near body temperature before being inserted in the eye.

It has been concluded that one of the sources of discomfort felt by users when inserting lenses into their eyes arises from the temperature of the lenses. In one arrangement, the disclosed tailored lens warming arrangements (or TLWA's) warm the contact lens to within a specified temperature range, this range being typically specified about a target temperature. This warming action, gently warming the lenses to the comfortable target range, preferably avoids inappropriate heating of the lens or the fluid in which the lens is stored.

The warming can be performed by applying a specified warming cycle, using a special-purpose lens warming apparatus, to the containers in which lenses are typically stored in a sterilizing fluid. This is done prior to insertion of the lenses into the wearer's eyes. This brings the lenses from an initial temperature (typically down to 16°C or lower depending on the ambient climatic temperature and domicile heating/insulation arrangements, and whether the lenses are stored in a refrigerator to reduce the growth of bacteria in the fluid) to within the specified temperature range, this being specified about the target temperature. The specified temperature range is preferably dependent upon the temperature of the exposed surface of the eye which is several degrees below body temperature (nominally 36.8°C), and possibly also dependent upon the ambient temperature.

The warming cycle can ensure, if desired, in bringing the lenses to the specified temperature range, (a) that during a "warm-up mode" the temperature of the lenses and the sterilizing fluid in which the lenses are stored remain within a rated specified temperature range, and/or (b) that during the warm-up mode neither the lenses being warmed, nor any part of the TLWA apparatus, exceed a specified maximum temperature, thus avoiding possible injury or discomfort to the user.

In one arrangement, the TLWA is used with removably insertable (also referred to as removably engageable) lens containers. In regard to this arrangement, there can be a number of different types and shapes of lens container. Repeatability and speed of the warming cycle in this arrangement can be facilitated by incorporating into the TLWA a heatable cavity shaped to be conformal to a corresponding contact surface of the particular lens container in question. This arrangement provides repeatable intimate contact over a substantial portion of the lens container between the heatable surface of the TLWA and the inserted mated lens container. This approach of using conformal cavities is applicable both to the TLWA arrangements discussed here, and to the TLWDA and bare dryer arrangements described below.

This intimate contact over a substantial contact area between the TLWA apparatus and the mated insertable lens container enables reliable rapid and repeatable warming of the lenses. Similar comments apply to the benefits of using intimate conformal surfaces in the drying arrangements. The associated warming cycle can take into account the thermal inertia of the lens containers, the heat transfer characteristics of the conformal interface between the TLWA apparatus and the inserted lens container, the size and mass of the lens container, the amount of sterilizing fluid in the container, the mass of the lenses and so on. The parameters associated with the warming cycle can be determined empirically, or analytically. Similar comments apply to the benefits of using intimate conformal surfaces in the drying arrangements.

Having regard to the variety of lens containers on the market, a corresponding variety of TLWA devices can thus be provided in order to provide the above-noted conformal interface between a given type of lens container and the corresponding warming apparatus. Each particular TLWA device can thus be tailored to provide the required conformal interface between the TLWA apparatus and the corresponding type of lens container. Similar comments apply to the benefits of using intimate conformal surfaces in the drying arrangements.

In an alternate arrangement, the lens container is not removably insertable into the TLWA, but it instead an integral part of the TLWA.

In yet another alternate arrangement, instead of providing a variety of TLWA devices in order to provide the conformal interfaces for different corresponding lens containers, a two-component TLWA apparatus can be used. In this arrangement one component of the TLWA apparatus is a heater module, and the other component is a shell which can accommodate a particular type of lens container. The shell can be mated to the heater module thus enabling operation of the TLWA apparatus as previously described. According to this arrangement, a user who wishes to change the type of contact lens they use (and hence to typically change the shape of the lens container) can purchase a suitable shell for use with their current heater module, rather than acquire an entirely new TLWA device. This type of arrangement can be used either in regard to insertably removable lens containers, or in regard to integral lens containers.

The TLWA approach makes the lenses more comfortable to insert into the wearer's eyes, thus preserving the wearer's comfort level, and possibly reducing the likelihood of injury by reducing the likelihood that the wearer will flinch when inserting an unwarmed lens into their eye. The TLWA approach can also help to ensure that the lenses and the sterilizing fluid in which the lenses are stored in the lens containers remain within their specified operating temperature range, thus also helping to maintain sterility of the lenses until they are removed from the lens containers.

The TLWA approach enables lens containers to be stored in a refrigerator, and the lenses to then be inserted into the users eyes, without the discomfort that would otherwise arise from the differential temperature between the cooled lenses and the users eye surfaces. Lenses can be stored in this manner to reduce growth of microbes in the sterilizing fluid in which the lenses are immersed in the lens containers.

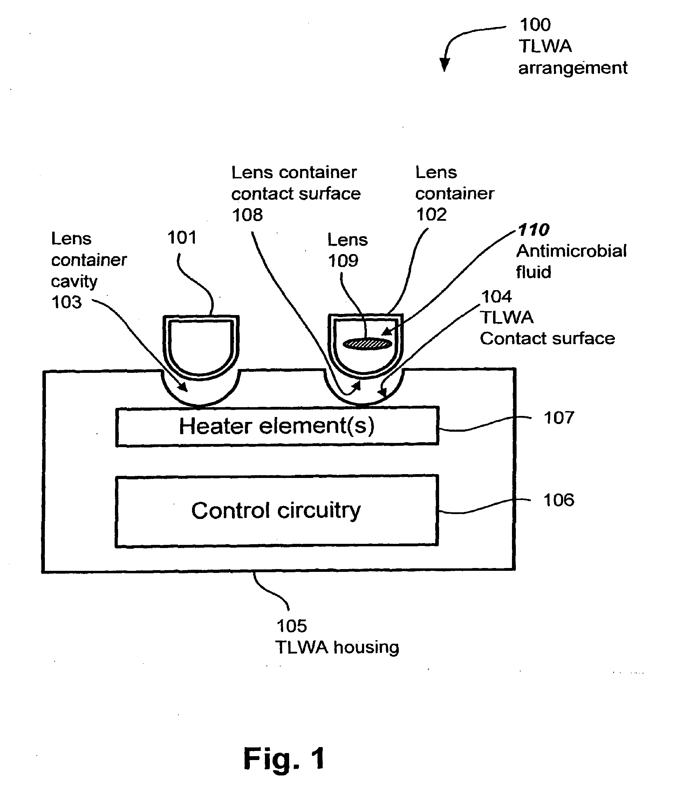

Fig. 1 shows a mechanical representation 100 of one example of a TLWA. The TLWA in this example comprises a housing 105 containing one or more heating elements 107 that are controlled by control circuitry 106, this also being referred to as a controller. In the described arrangement, contact lenses such as 109 are each sealed in a lens container 102 that typically contains 2-10 ml of sterilizing fluid 110. The TLWA housing 105 has cavities such as 103 each having a heatable contact surface 104 that is shaped to be conformal to a corresponding lens container contact surface 108. The cavities are referred to as heatable cavities. The intimate contact (ie mating) that is thus achieved between the conformal TLWA contact surface 104 and the lens container contact surface 108 when the lens container 102 is removably inserted (meaning that the lens container can be inserted into, or engaged with, the cavity, and then removed) into the lens container cavity 103 enables the heating elements 107 to rapidly, accurately and repeatably deliver the desired temperature/time warming profile. This profile ensures that the lens 109 is brought to within a specified temperature range about a desired target temperature. This is preferably achieved within a specified time interval. The TLWA arrangement also preferably maintains the lenses, after they have been warmed to the specified temperature range, within that range for a further specified time interval.

In one arrangement, the target temperature is specified to be 36°C. The specified temperature range about the target temperature can be specified as +/- 2°C about the target temperature or if circumstances so dictate, a greater range can be specified. In this arrangement, the specified time interval to reach the specified temperature range is 2 minutes +/- 30 seconds or if circumstances so dictate, a greater range can be specified. In this arrangement, during the warm-up mode, the TLWA ensures that neither the temperature of the TLWA apparatus nor any parts thereof, nor that of the lens container 102 or the lens 109 contained therein, overshoots (ie exceeds) a specified maximum temperature of 65°C. The specified time interval to maintain the temperature at the specified temperature range about the target temperature is 5 minutes +/- 2 minutes or if circumstances so dictate, a greater range can be specified.

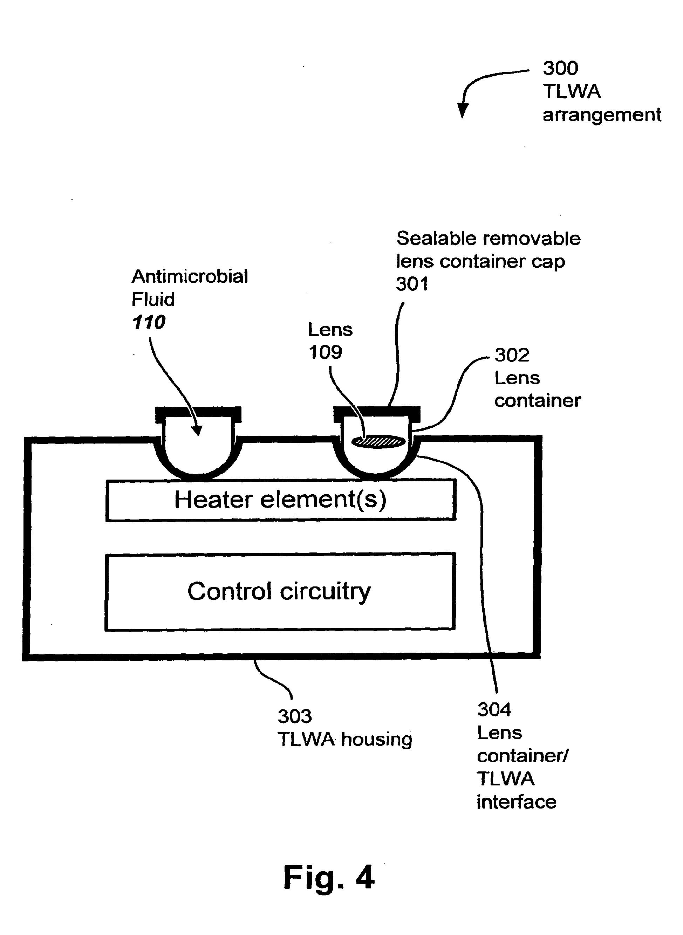

The lens container 102 can take different forms within the aforementioned description, to accommodate use with either one-time use disposable lenses or use with reusable lenses. From a terminology standpoint, a lens container can be considered to comprise a lid or cap 301 (see **Fig. 4**), and a container or container vessel 302.

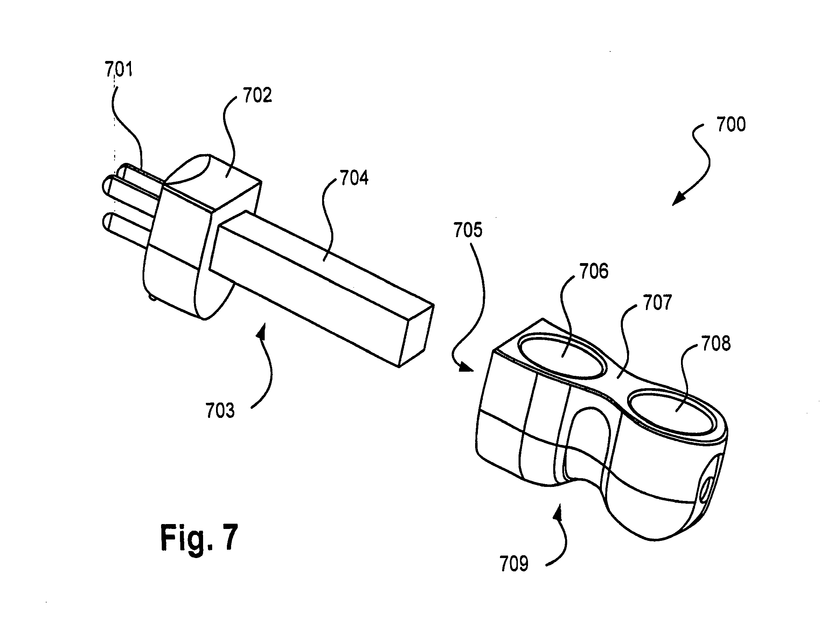

Different configurations of lens container 102 can be accommodated by providing associated TLWA devices with correspondingly configured conformal lens container cavities 103, or (b) by providing associated TLWA shells with correspondingly configured conformal lens container cavities, these shells being usable with a heater module (see **Fig. 7** for more details).. This "tailoring" of the TLWA cavities 103 to the lens containers 102 ensures intimate mating between the conformal TLWA contact surface 104 and the lens container contact surface 108 thus enabling the TLWA to operate in a repeatable manner irrespective of the particular configuration of the lens container 102. The lens wearer thus is able to use a TLWA device that is tailored to the particular configuration of lens container that he or she prefers.

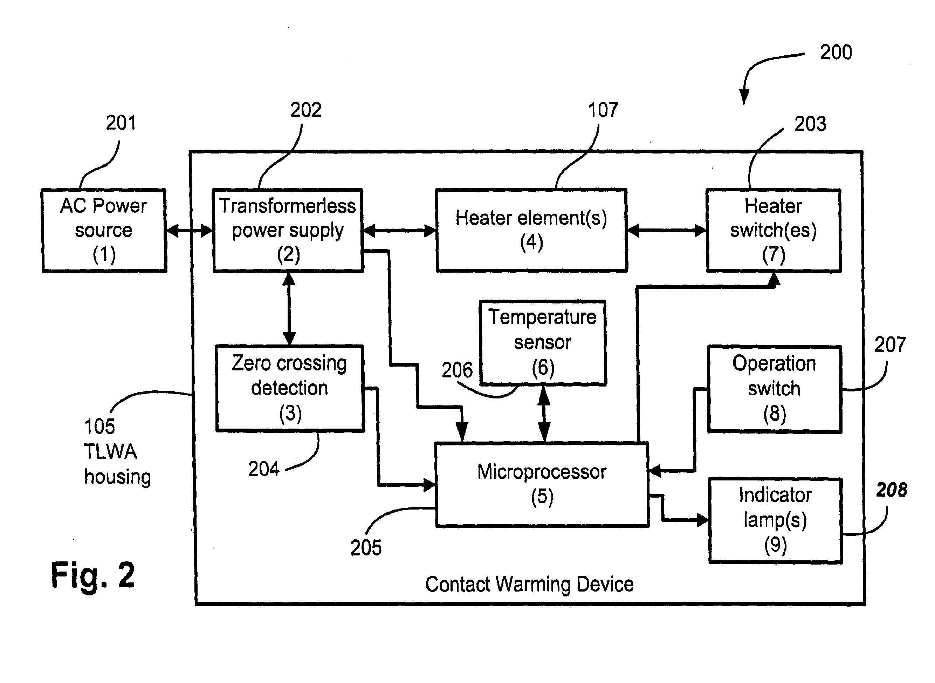

**Fig. 2** shows an electrical (control and heating) representation 200 of the TLWA of **Fig. 1****.** The TLWA 200 comprises the following components in the present example:
An external AC power source 201;
a transformerless power supply 202;
a zero-crossing detector 204;
a heater element 107;
a micro controller 205;
a temperature sensor 206;
a heater switch 203;
a system operating switch 207 (also referred to as a selector switch); and indicating lamps 208.

Power is supplied from the power source 201, via an AC electrical wall socket. The transformerless power supply parasitically taps a portion of the AC current provided by the AC power source 201 and converts it into a 5V DC power supply for the operation of the microprocessor 205, the temperature sensor 206, the heater switch 203, and the indicator lamps 208. The supply is "parasitic" in the sense that only a small amount of power (approximately 1/1000th of power normally available from the wall socket) is required for the control electronics and is derived from the main AC power supply. The zero crossing detector 204 detects the zero crossing point of the AC current in the main AC power supply and this zero crossing point is used to time the operation of the heater switches 203 in order to minimise the electrical noise associated with the operation of the heater switches 203. The heater elements 107 are controlled, via the heater switches 203, by the micro-controller 205. The temperature sensor 206 monitors the temperature of the interface between the TLWA contact surface 104 and the lens container contact surface 108 and provides a feedback signal to the micro-controller 205. The operating switch 207 enables an external input from the user to be used to switch the TLWA 200 from an inactive to an active state. The indicating lamps 208 indicate the state of operation of the TLWA device 200.

When the TLWA device 200 is activated via the operating switch 207, in one arrangement the temperature of the heating elements 107 temperature is raised to a preset temperature for the period necessary to overcome the thermal inertia of the TLWA device 200 and the lens container 102 containing the contact lens 109. Once the aforementioned thermal inertia is overcome, the temperature sensor 206 is employed to monitor the TLWA device temperature at the interface between the TLWA contact surface 104 and the lens container contact surface 108. The temperature sensor 206 provides feedback to the microcontroller 205 so that the micro-controller 205 can control the heater elements 107 to bring the temperature of the contact lens container 102 to within the specified temperature range.

As previously noted, various warming profiles can be used, controlled by various control algorithms, provided that the desired temperature/time profile is satisfied. Thus, for example, on-off, proportional, proportional-integral-derivative (PID) or other control algorithm making use of the temperature sensor throughout the entire warm-up mode (see below) can be used.

Although the temperature sensor 206 in the described arrangement monitors the TLWA device temperature at the interface between the TLWA contact surface 104 and the lens container contact surface 108, other sensor arrangements can be used provided that the required temperature/time profile is satisfied. Thus, for example, an alternate arrangement can utilize one or more temperature sensors that monitor the temperature of the heater elements. This temperature sensor arrangement in conjunction with an algorithm on the microcontroller can be used to extrapolate the temperature of the interface based on characterization of the heater elements, the TLWA case and the lens container.

The disclosed TLWA arrangements support 3 modes of operation:
1. **Standby mode** - in which the TLWA device is neither warming nor maintaining the temperature of the lens container 102 (the TLWA device being either completely disabled, or in a state where some components are operating in order to reduce the start-up time when the TLWA device enters the next mode);
2. **Warm-up mode** - in which the TLWA device is raising the temperature of the TLWA contact surface 104, and by extension, the temperature of the contact lens container 102 from storage temperature to within the specified temperature range, preferably within the specified time interval without overshooting the specified maximum temperature; and
3. **Maintain temperature mode** - in which the temperature of the TLWA contact surface 104 is monitored by the temperature sensor 206 and controlled in order to maintain the temperature of the lenses within the specified temperature range for the specified time interval. When the set period expires the device returns to standby mode.

When the system operation switch 207 is operated once, the TLWA device automatically cycles through the three above-noted operational modes in sequence. The operational state of the TLWA is indicated via the indicator lamp 208. The colour of the indicator lamp can be varied (using either a multi-coloured LED or multiple LED's for example) to indicate the state of the TLWA device.

Although **Fig. 2** depicts one type of control arrangement, other arrangements can be used within the scope of the TLWA concept. Thus for example the TLWA apparatus can be configured as a "plug pack" which is plugged directly into the AC power socket. In this arrangement, the system operation switch 207 can be omitted, and the system can be activated by the insertion of the plug pack into the power socket, this automatically causing the TLWA apparatus to cycle through the operational modes described. In another arrangement, the AC power source 201 and the power rectifier 202 can be replaced by a DC power source and suitable voltage regulator respectively. Alternately, an internal battery can be incorporated into the TLWA device in addition to or in place of the external power arrangement, thus increasing the portability of the TLWA arrangement. Furthermore, the indication lamps 208 can be omitted, if desired, or replaced with LCD indicators.

Furthermore, as previously noted that the control algorithm used by the microcontroller 205 to control the heater switches 203 and consequently the heater elements 107 can be based upon on-off, proportional, PID or other control methodologies, provided that the desired time/temperature profile can be achieved.

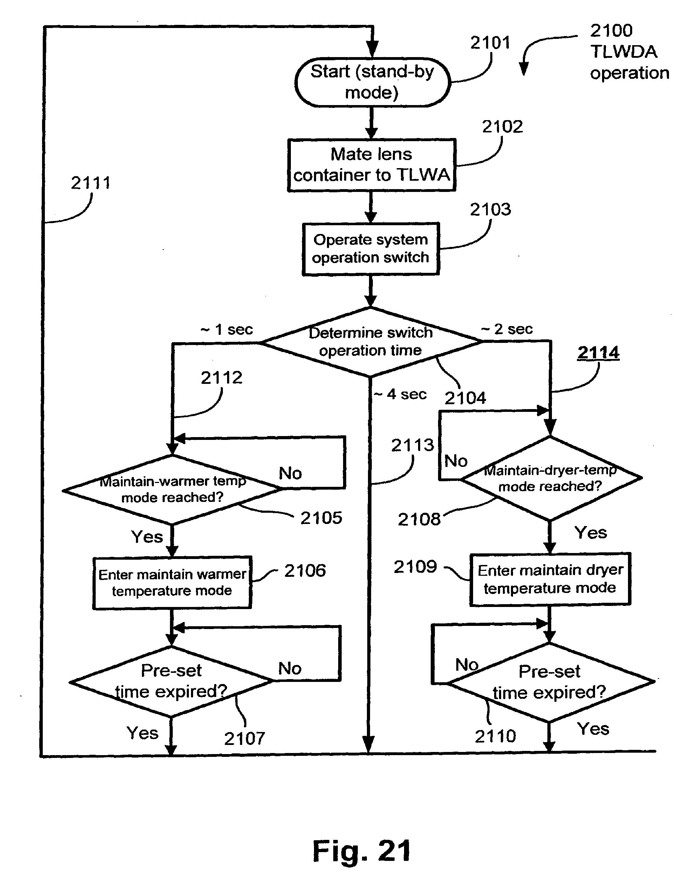

Although the electrical arrangement depicted in **Fig. 2** has been described with reference to the TLWA arrangement of **Fig. 1****,** the arrangement in **Fig. 2** can also be used with the TLWA arrangements depicted in **Figs. 4****,** **7** **and** **8****.** Furthermore, the TLWDA arrangements described below can use similar arrangements to those described above in regard to the TLWA. As will be described in regard to **Fig. 21****,** the above **standby, warmup,** and **maintain-temperature** modes are used, with appropriate timing and temperature parameters and profiles, in the warming mode and the drying mode, as determined by the amount of time that the user operates (eg presses) the system operation switch 207.

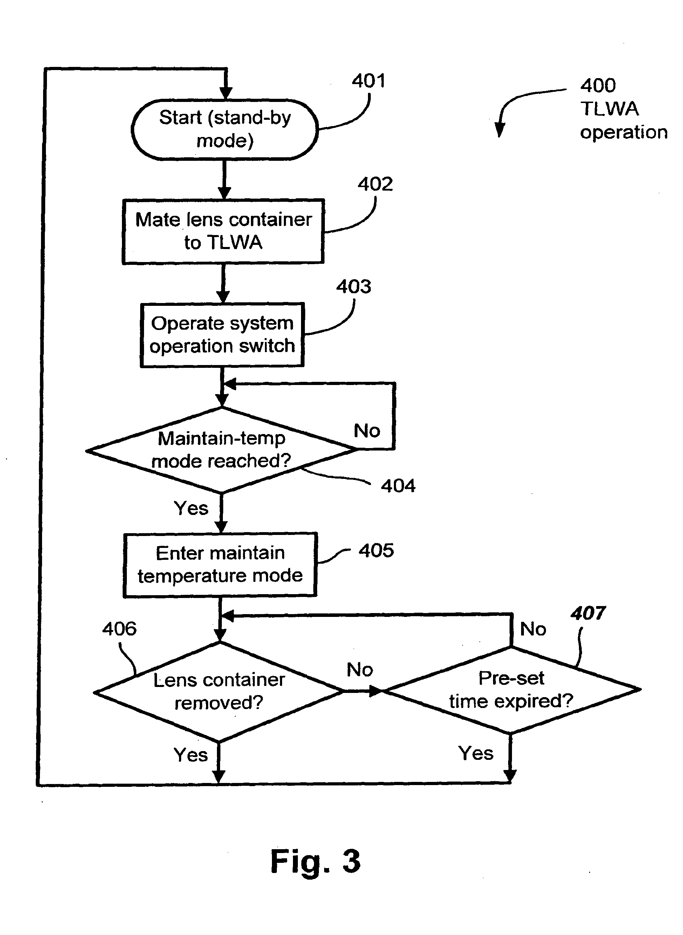

**Fig. 3** shows a process 400 of how the disclosed TLWA device would typically be operated. In the example shown the process commences with a step 401 in which the TLWA device is in the "Stand-by mode". In a following step 402 the user removably inserts the lens container 102 to the TLWA device cavity 103 ensuring mating (ie conformal contact) between the lens container contact surface 108 and the TLWA contact surface 104. In a subsequent step 403 the user operates the system operation switch 207, thereby providing a system actuation signal initiating a device safety check, and subsequently initiating the "Warm-up mode". A subsequent decision step 404 determines if the TLWA device has reached the "Maintain temperature mode", as would be indicated by the lamp 208. If this is not the case, then the process 400 follows a NO arrow back to the step 404 in a looping fashion. If the step 404 indicates, according to the temperature sensor 206, that the lens container 102 has reached the "Maintain temperature mode" (this occurring when the lens container reaches the specified temperature range), then the process 400 follows a YES arrow to a step 405 in which the micro-controller 205 switches the TLWA device into the "Maintain temperature mode".

A following step 406 determines whether the lens container 102 has been removed from the lens container cavity 103 by sensing a slight change in the temperature of the interface between the lens container and the TLWA apparatus. If this is not the case, then the process follows a NO arrow to a step 407. The step 406 is optional and may be omitted as desired in alternate implemenations. The step 407 determines if a pre-determined maintenance time interval has expired If this is not the case, then the process 400 follows a NO arrow back to the step 406. As noted, when the user removes the lens container 102 from the cavity 103, the temperature sensor 206 detects a temperature change, the step 406 returns a logical TRUE, and the process 400 follows a YES arrow according to which the micro-controller 205 returns the TLWA device to the "Standby-mode" in the step 401.

Returning to the step 407 for a functional description thereof, if the user does not remove the lens container 102 from the container cavity 103, the TLWA device maintains the temperature of the lens container 102 within the specified temperature range until the preset timer in the micro controller 205 has expired, in which event the micro-controller 205 returns the process 400 to the step 401 which places the TLWA device in the "standby mode". The maintenance period of 5 minutes can be varied, having regard to the fact that bacteria can begin to grow in the fluid in the lens container if this time becomes extended. The described arrangement allows a window of opportunity defined by the specified maintenance time for the user to remove the container 102, and if such does not occur, the TLWA device then shuts down to save power.

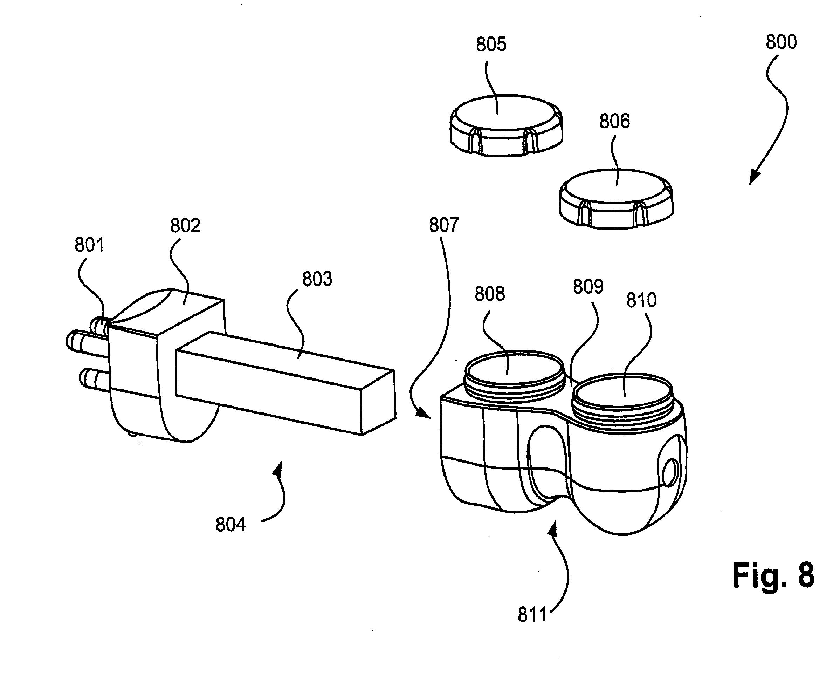

Although the process 400 in **Fig. 3** has been described with reference to the TLWA arrangement of **Fig. 1****,** it can also be used with the TLWA arrangement depicted in **Figs. 7** and **8****.** The steps 403-405 in the process 400 apply to the TLWA arrangements depicted in **Figs. 4** and **8****.** These TLWA arrangements can, in a step similar to the step 406, detect when the lenses are removed from the respective integrally formed lens containers, after which the TLWA arrangements re-enter stand-by mode after expiration of a suitable pre-set time interval.

**Fig. 4** shows a mechanical representation 300 of another example of the TLWA. In this arrangement a lens container 302 is an integral (non-removable) part of the TLWA housing 303. Accordingly, in one arrangement the container 302 can be formed as a cavity in the housing 303 with an integral collar projecting from the housing to enable the cap 301 to be fitted to the resultant "container". In another arrangement, the container can be formed by inserting a separate container in a non-removable fashion into a cavity in the housing, eg by press-fitting the container so that it becomes "integral" with the housing.

The lens container in this example has a removable cap 301 which is removed in order to remove the lens 109 from the container 302. According to the second arrangement noted above, the mating between the (separate) lens container 302 and the TLWA device is permanent, and forms a permanent lens container / TLWA interface 304.

Operation of the TLWA device in **Fig. 4** **is** similar to that of the arrangement in **Fig. 1** except that there are additional steps for maintaining the sterility of the contact lens whilst it is in the TLWA device. Thus, for example, it would be necessary to clean the lens container 302 and the removable cap 301 using suitable cleaning materials between successive uses of the TLWA arrangement. The step 406 in **Fig. 3** may be omitted in this arrangement if the change in mass is too small to be reliably detected.

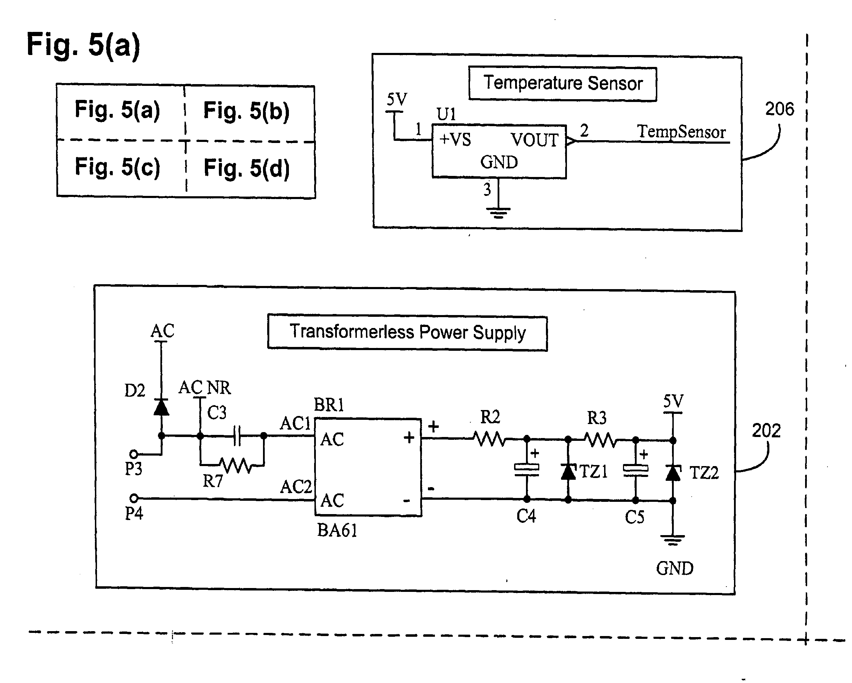

**Figs. 5(a) - 5(d)** show one example of a schematic diagram for the electrical (control) aspects of the TLWA of **Fig. 1****.** Schematic sub-systems for the heater elements, heater switches, zero crossing detection, temperature sensor, transformerless power supply, operational switch, microprocessor and indicator lamps are designated by respective reference numerals 107, 203, 204, 206, 202, 207, 205and 208 respectively.

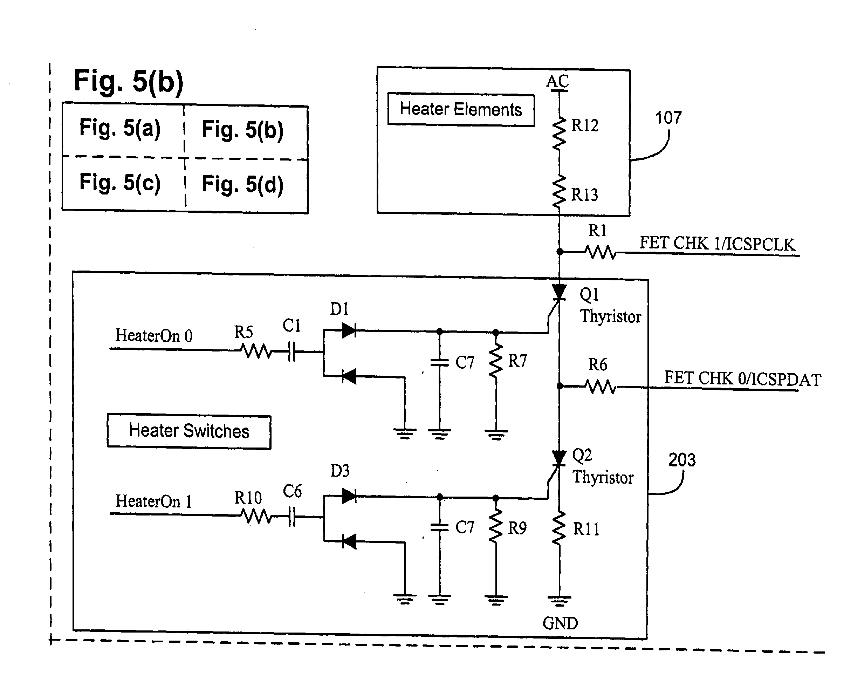

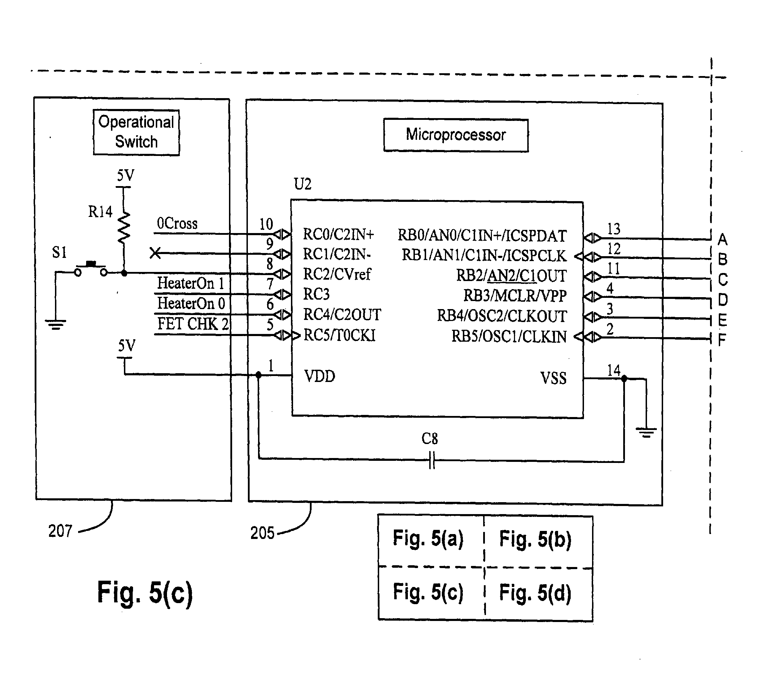

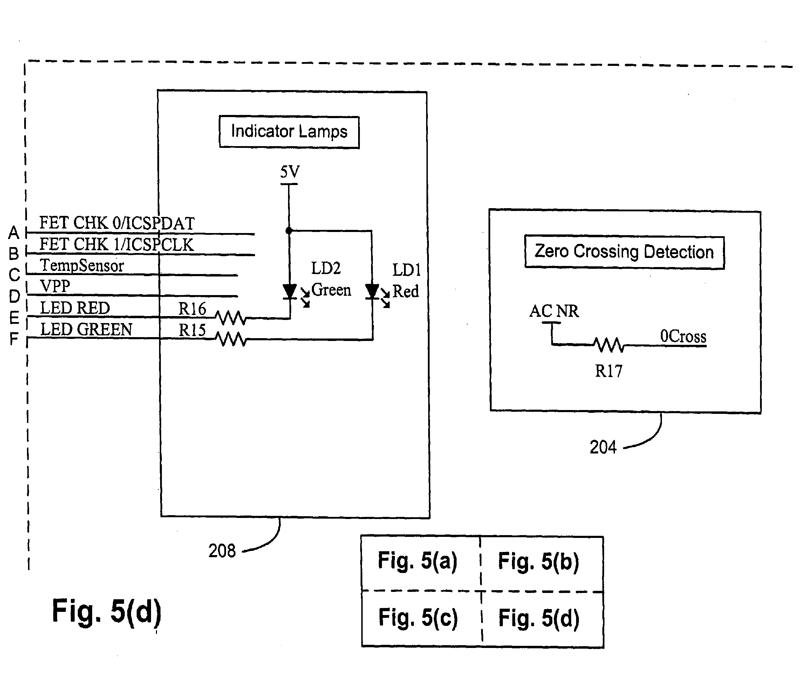

Although the electrical arrangement depicted in **Fig. 5** has been described with reference to the TLWA arrangement of **Fig. 1****,** the arrangement in **Fig. 5** can also be used with the TLWS arrangements depicted in **Figs. 4****,** **7** and **8****.**

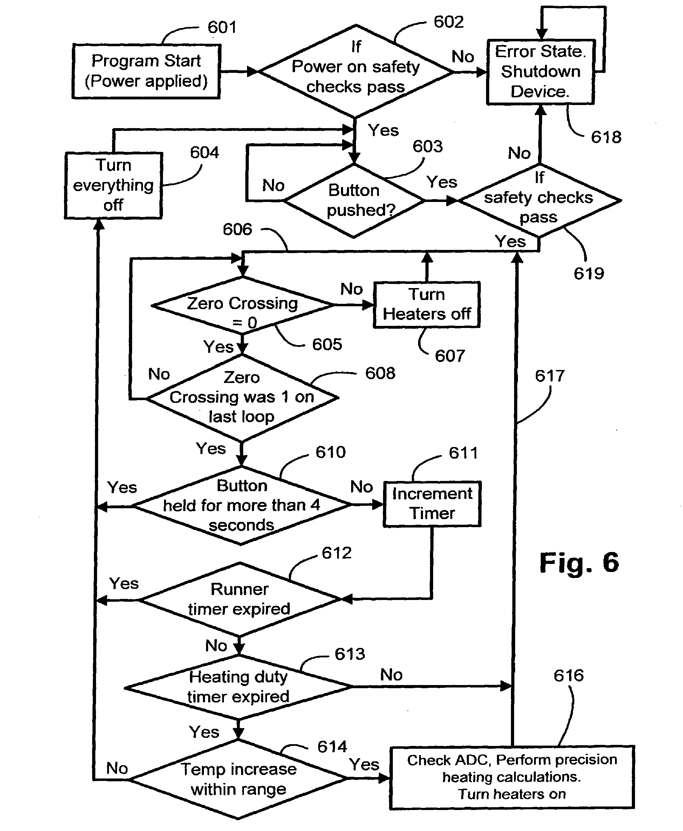

**Fig. 6** shows one example of a flow chart depicting a process 600 that can be used by the TLWA controller. **APPENDIX A** sets out a pseudo-code implementation of how the aforementioned process 600 can be implemented. The process 600 commences with a start step 601 in which power is supplied, after which a decision step 602 determines if the power safety check being conducted by the microprocessor 205 has been passed. If this is not the case then the process follows a NO arrow to a step 618, which constitutes an "error state" and the TLWA shuts down. If however the step 602 returns a logical TRUE, the process follows a YES arrow from the step 602 to a decision step 603. In the step 603 the microprocessor 205 determines if the button 207 has been activated. If this is not the case, then the process 600 follows a NO arrow back to the step 603 in a looping fashion. If the step 603 returns a logical TRUE then the process follows a YES arrow to a decision step 619 which conducts a further determination of whether safety checks have been passed. If this is not the case, then the process 600 follows a NO arrow from the step 619 to the step 618. If however the step 619 returns a logical TRUE, then the process 600 follows a YES arrow from the step 619 to a decision step 605 in which the microprocessor 205 and the zero crossing detection module 204 determine if the zero crossing detection is a "0" (ie at approximately 0 volts). If this is not the case then the process follows a NO arrow to a step 607 in which the microprocessor 205 turns the heater elements 107 off using the heater switches 203. The process is then directed as depicted by an arrow 606 back to the step 605.

If the step 605 returns a logical TRUE then the process follows a YES arrow to a step 608 in which the microprocessor 205 and the zero detection module 204 determine if the zero crossing was "1" (ie greater than 0 volts) on the last loop. If this is the case, then the process follows a YES arrow to a step 610 in which the microprocessor 205 determines if the button 207 was held for more than 4 seconds. If this is the case, then the process follows a YES arrow to a step 604 in which the microprocessor 205 turns the TLWA off. The process if then directed by an arrow 615 to the step 603.

Returning to the step 608, if the step returns a logical FALSE, then the process follows a NO arrow to the step 605. Returning to the step 610, if the step returns a logical FALSE, then the process follows a NO arrow to a step 611 in which the microprocessor 205 increments a timer relating to the maintenance time. In a following decision step 612 the microprocessor 205 determines if the timer has expired. If this is the case, then the process is directed by a YES arrow to the step 604.

Returning to the step 612, if the step returns a logical FALSE, then the process follows a NO arrow to a step 613 in which the microprocessor 205 determines if the warming duty timer has expired. If this is not the case, then the process follows a NO arrow back to the step 605. If however the step 613 returns a logical TRUE then the process follows a YES arrow to a step 614 in which the microprocessor 205 determines if the temperature increase is within range. The temperature range referred to here relates to the maximum allowable temperature, the maximum allowable rate of change in temperature for the entire system, and where the current temperature fits within these operational parameters. If this is not the case, then the process follows a NO arrow to the step 604. If however the step 614 returns a logical TRUE, then the process follows a YES arrow to a step 616 in which the microprocessor 205 checks the Analogue to Digital Conversion (ADC), performs precision warming calculations, turns the heating elements 107 on using the heating switches 203, and the process follows an arrow 617 back to the step 605.

**Fig. 7** shows a mechanical representation of an example of a two-component TLWA. The TLWA in this example comprises a first component being a heater module 703, and a second component being a shell 709, these two components forming the two-component TLWA when thermally connected.

The depicted heater module 703 has three pins 701 which are adapted (i.e. configured) for insertion into a standard power socket. Clearly other pin arrangements can be used, such as two pin configurations which do not have an earth pin. The heater module 703 also has a housing 702 which can contain the electronic circuitry shown in **Fig. 5****.** The heating element 704 extends from the housing 702 and is shaped for insertion, as depicted by an arrow 705, into a correspondingly shaped socket (not shown) in the shell 709.

The heater module and the shell are thermally connectable by shaping the heating element and the socket in a manner as to ensure snug contact (i.e. good thermal contact) between the heating element 704 and the shell 709 when the heating element is fully inserted into the socket. This full insertion also enables operation of the operational switch S1 at 207 (see **Fig. 5**) thereby providing a safety interlock which prevents the heater element 704 from heating up while outside the socket in the shell 709.

The shell 709 comprises a shell housing 707 and, in the example shown in **Fig. 7****,** two lens container cavities 706 and 708 formed in the shell housing 707. These lens container cavities 706 and 708 are tailored to each have a heatable contact surface (such as 103 in **Fig. 1**) that is shaped to be conformal to a corresponding lens container contact surface (such as 108 in **Fig. 1**). This two-component arrangement enables a user to keep the heater module 703 and merely change the shell 709 if the user wishes to change the lens supplier and hence change the shape of the lens containers.

The shell 709 is typically made of a material whose thermal conduction properties facilitate heating of the lens containers (not shown) when inserted into the respective lens container cavities 706, 708.

Although the TLWA arrangement in **Fig. 7** shows the heater module 703 adapted for insertion into a socket in the shell 709, other arrangements can be used to mate (i.e. thermally connect) the heater module and the shell in order to provide suitable thermal contact. Thus, for example, the heater module could consist of a flat heating plate 710 adapted for pressure mating against a corresponding flat surface (not shown) at 711 on the shell 709.

Furthermore, although the TLWA arrangement in **Fig. 7** depicts a particular configuration of shell which completely envelops the heating element 704 when the shell 709 and the heating module 703 are thermally connected, other two-component TLWA arrangements can be used.

**Fig. 8** shows a mechanical representation of another example of a two-component TLWA. The TLWA in this example comprises a heater module 804 and a shell 811. The depicted heater module 804 has three pins 801 which are adapted for insertion into a standard power socket. Other pin configurations, including two pin arrangements which do not include an earth pin, can also be used. The heater module 804 also has a housing 802 which can contain the electronic circuitry shown in **Fig. 5****.** The heating element 803 extends from the housing 802 and is shaped for insertion, as depicted by an arrow 807, into a correspondingly shaped socket (not shown) in the shell 811.

The heating element and the socket are shaped in a manner as to ensure good thermal contact between the heating element 803 and the shell 811 when the heating element is fully inserted into the socket. This full insertion also enables operation of the operational switch S1 at 207 (see **Fig. 5**) thereby providing a safety interlock which prevents the heater element 803 from heating up while outside the socket in the shell 811.

The shell 709 comprises a shell housing 809 and, in the example shown in **Fig. 8****,** two integrally formed lens cavities 808 and 810. These lens cavities 808 and 810 may be of any convenient shape for enabling contact lenses to be stored therein in a similar manner to that depicted in **Fig. 4****.**

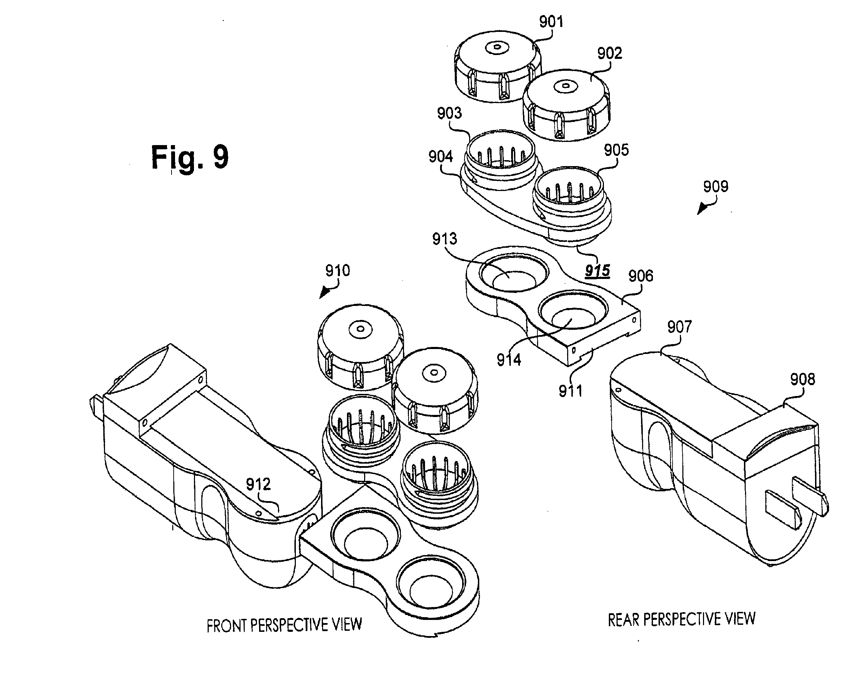

**Fig. 9** shows a mechanical exploded representation of a third example of a two-component TLWA together with lens container lids and vessels.

One view 909 shows a rear perspective view of a heating module 908 having a dovetailed heat conductive rib 907 to which a removably engageable housing 906 can be engaged. A complimentary dovetailed channel 911 in the under side of the housing 906 can be engaged with the dovetailed rib 907 to enable the heating module 908 to efficiently transfer heat to the housing 906. The housing 906 has heatable cavities 913, 914 that are shaped to conform to a lower external surface 915 of container vessels 905, 903 that, in the present example, are permanently mounted to a vessel mounting plate 904. The container vessels 905, 903 have associated container lids 902, 901. A view 910 shows a front perspective view of the elements shown in the rear perspective view 909. In some of the following figures the containers referred to in the figures are referred to by reference numerals associated either with the container lids, or the container vessels. The intended meaning is clear in light of the context.

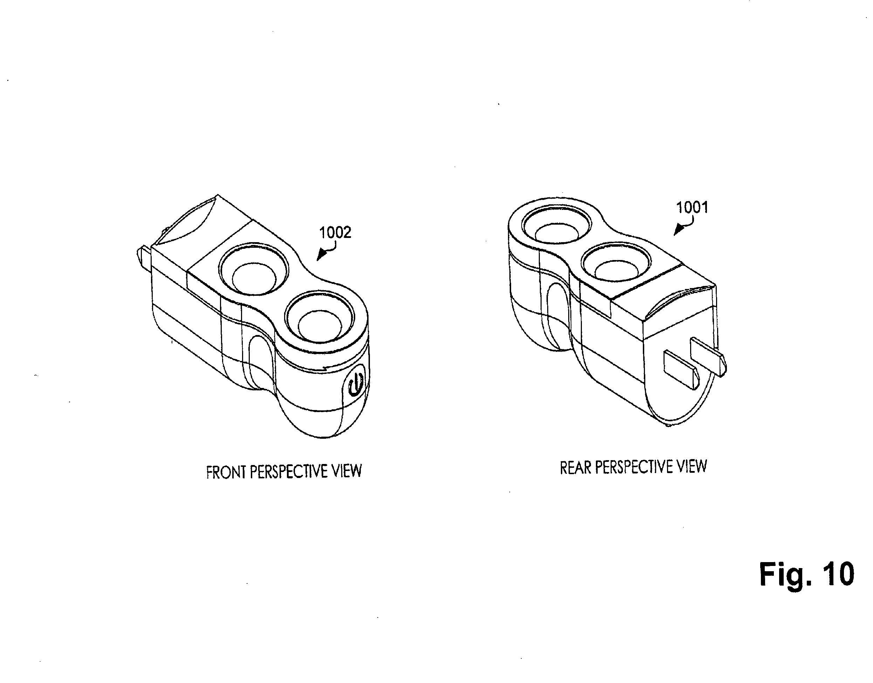

**Fig. 10** shows a rear perspective view 1001 and a front perspective view 1002 of the TLWA of **Fig. 9** in assembled form without the lens containers.

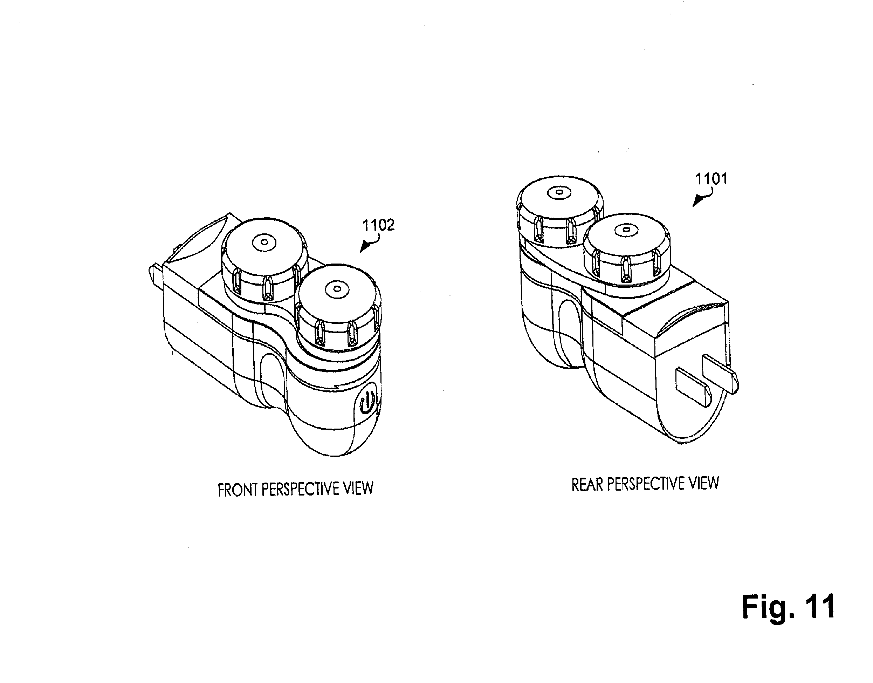

**Fig. 11** shows a rear perspective view 1101 and a front perspective view 1102 of the TLWA of **Fig. 9** together with the lens containers that have been engaged with the housing 906.

### Tailored Lens Warming and Drying Arrangements (TLWDA)

There is a significant increase in the risk of eye infection for contact lens wearers if the contact lens storage container is not dried properly between uses.

Storage containers, left open within a room, may become contaminated with airborne dust, microbes and mould spores. Accordingly, the TLWDA arrangements configure the storage container and lids while they are being dried in a manner that shields the containers from ingress of airborne particles, dust and mould spores during the drying process and subsequent storage period between uses.

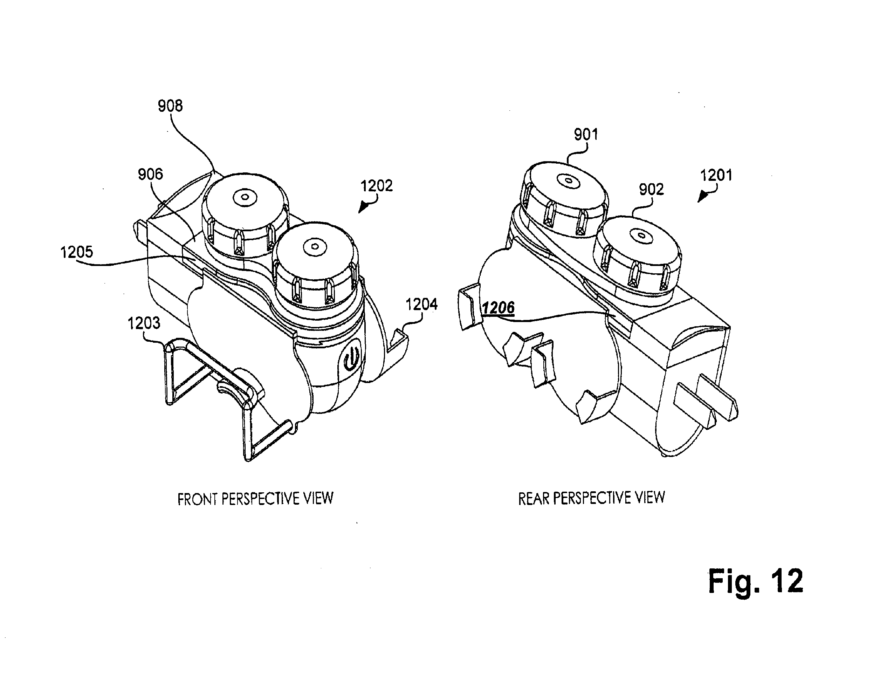

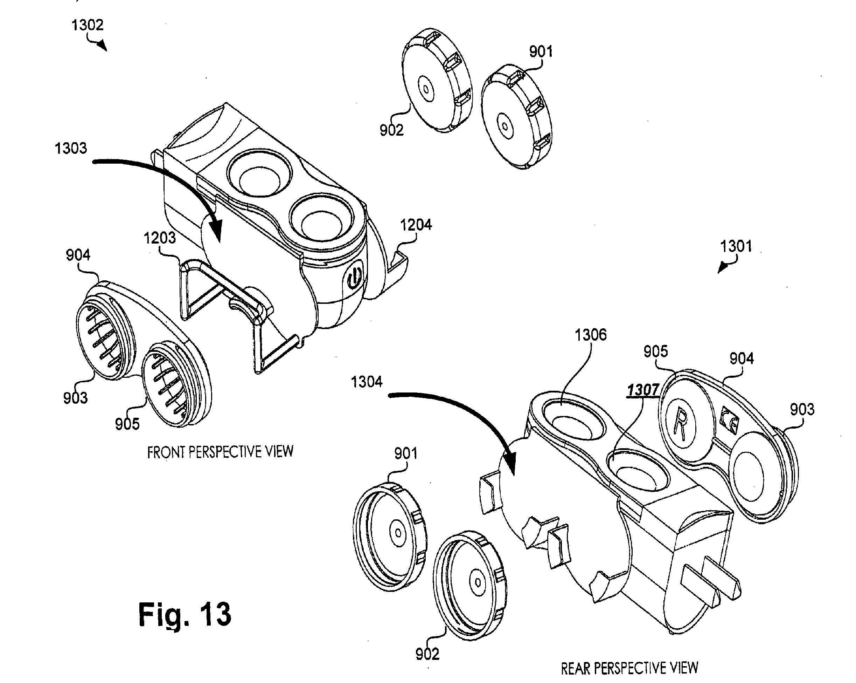

**Fig. 12** shows a mechanical representation of a first example of a two-component TLWDA, with lens containers mounted in a warming configuration for warming. A front perspective view 1202 shows the heating module 908 and the removably engaged contact lens container housing module 906. The housing 906 in the present arrangement has drying engagement mechanisms or clips 1203, 1204 affixed to the respective edges 1205, 1206 of the housing 906. This enables the heating module 908 to transfer heat to the containers 901 and 902 when the housing 906 is mated to the heating module 908 as shown. The containers 901 and 902 are depicted as being removably engaged in their respective heatable cavities 1306, 1307 (see **Fig. 13**). In this first example, the presence of the containers 901, 902 does not obstruct the drying engagement mechanisms or clips 1203, 1204. Accordingly, the TLWDA can be used in warming mode with the containers 901, 902 engaged with their respective warming cavities 1306, 1307. The TLWDA can also be used in drying mode with the containers 901, 902 engaged with their respective drying engagement clips 1203, 1204 as will be described in relation to **Fig. 13****.**

**Fig. 13** shows the two-component TLWDA of **Fig. 12** with lens container lids and vessels oriented for drying. A front perspective view shows the container vessels 905, 903 permanently mounted to the vessel mounting plate 904 oriented for engagement with the drying clip 1203. In the front perspective view 1302 it can be seen how the vessel/plate assembly 903-905 is inserted or engaged, as depicted by an arrow 1303, into the drying clip 1203. In the rear perspective view 1301 it can be seen how the lids 901, 902 are inserted or engaged, as depicted by an arrow 1304, into the drying clip 1204. Importantly, the vertical orientation of the vessel/plate assembly 903-905 and the lids, when engaged with their respective drying clips 1203, 1204, cause the respective side walls of the vessels and the lids to shield the internal cavities of the vessels and the lids, thus reducing the gravity ingress of airborne contaminants into the vessels during the drying cycle.

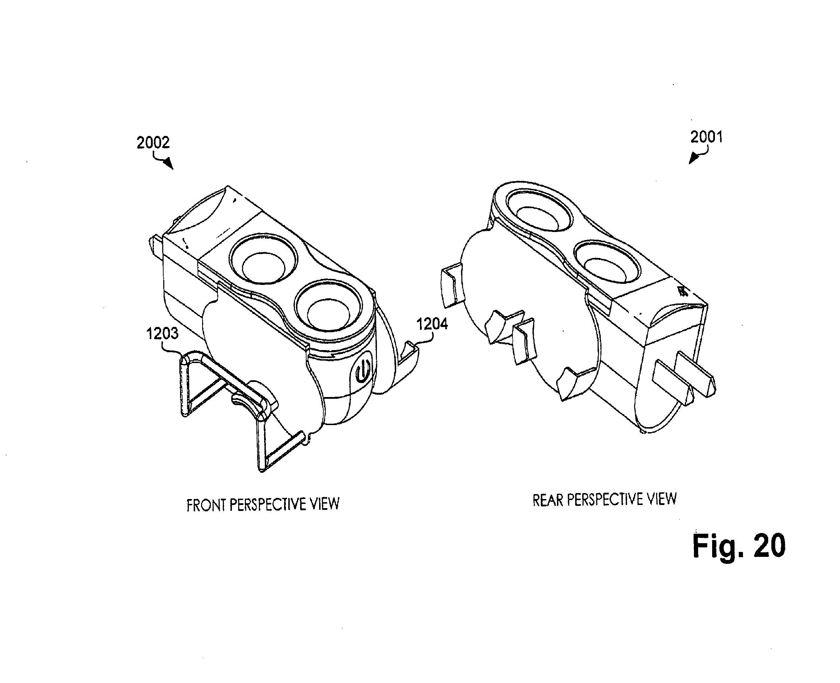

**Fig. 20** shows the TLWDA of **Fig. 12** without lens containers.

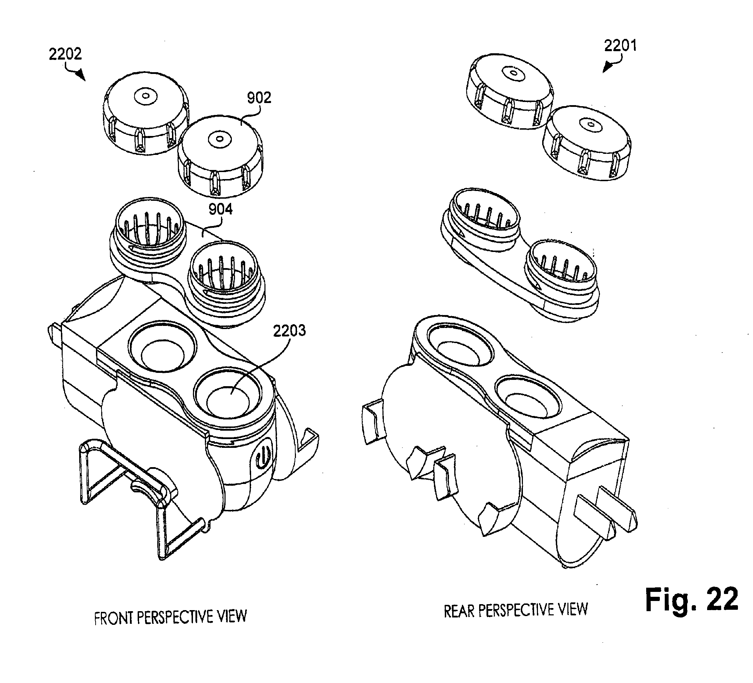

**Fig. 22** shows the two-component TLWDA of **Fig. 12** with lens container lids and vessels oriented for warming.

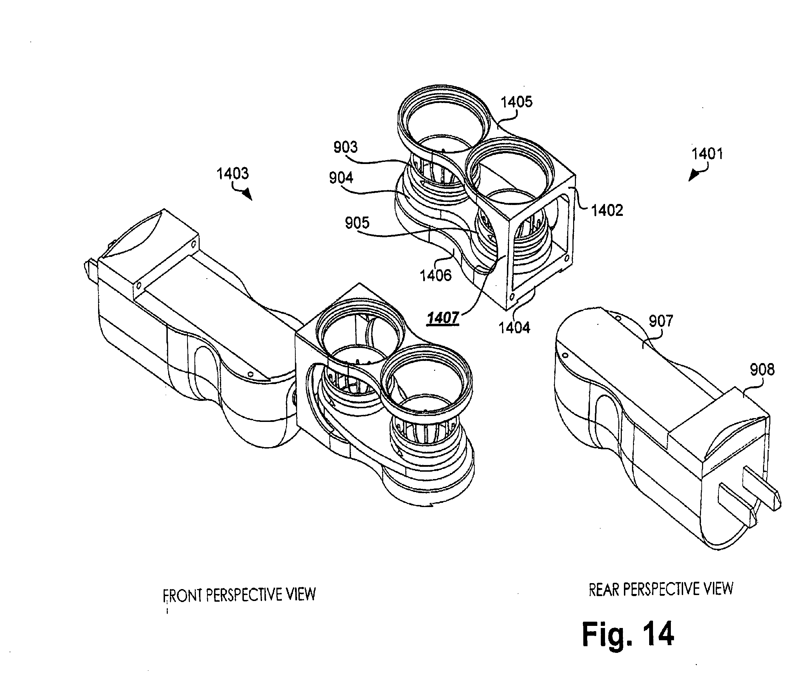

**Fig. 14** shows an exploded mechanical representation of a second example of a two-component TLWDA with lens container vessels mounted for warming or drying. One view 1401 shows a rear perspective view of the heating module 908 having the dovetailed heat conductive rib 907 to which a removably engageable housing 1402 can be engaged. A view 1403 shows a front perspective view of the heating module 908 and the dovetailed heat conductive rib 907 to which the removably engageable housing 1402 can be engaged.

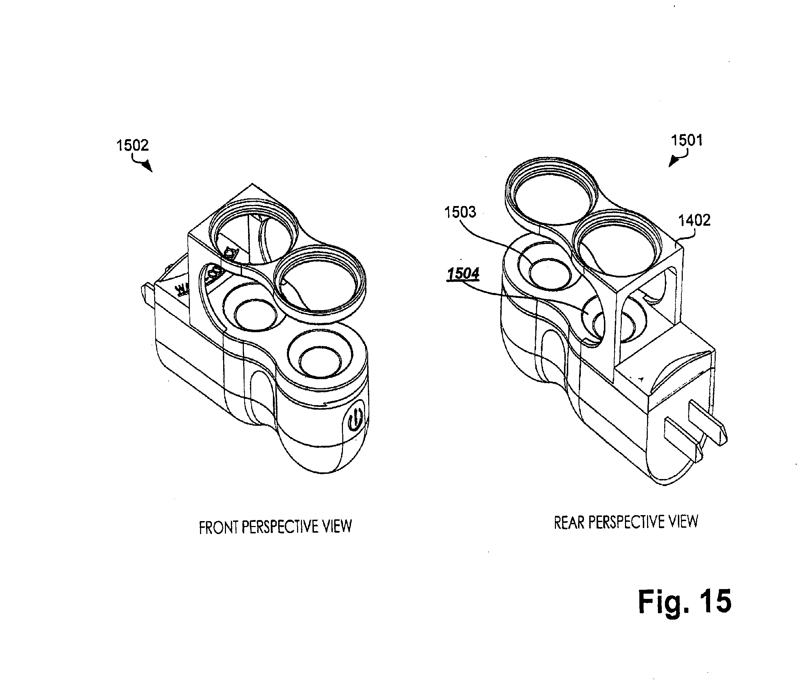

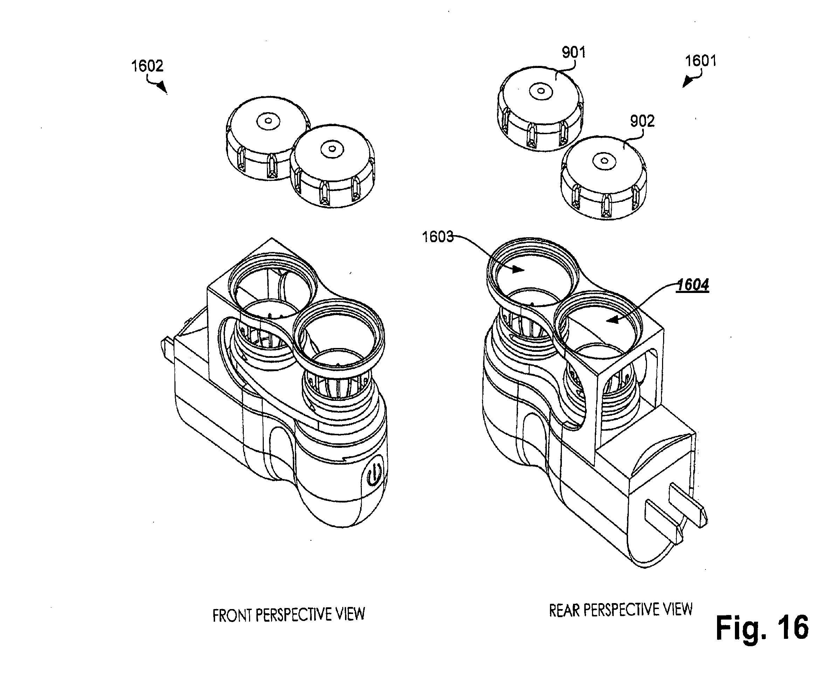

A complimentary dovetailed channel 1404 in the under side of the housing 1402 can be engaged with the dovetailed rib 907 to enable the heating module 908 to efficiently transfer heat to the housing 1402, thus enabling the heating module to perform the desired one of the heating and drying operations. The vessel/plate assembly 903-905 is engaged with respective heatable cavities 1503, 1504 (see **Fig. 15**). The removably engageable housing 1402 in this example comprises a lower plate 1406 that is similar to the housing 906 in **Fig. 12****.** However, the housing 1402 has an upper plate 1405 as well, which is joined to the lower plate 1406 by vertical brackets 1407. The physical arrangement in this example enables the containers depicted by the vessel/plate assembly 903-905 to be engaged to the same heatable cavities 1503, 1504 for both the warming mode and the drying mode. This eliminates the need to move the containers from the heating cavities 1306, 1307 to the drying clips 1203, 1204 as depicted in **Fig. 13****.** The container lids 901, 902 (see **Fig. 16**) can be secured to the containers depicted by the vessel/plate assembly 903-905 if the warming mode is to be used. Alternately, The container lids 901, 902 can be positioned on the respective lid drying supports 1603, 1604 (see **Fig. 16**) if the drying mode is to be used. Importantly, in the drying mode the lids 901, 902, when supported on the lid drying supports 1603, 1604, shield the vessels and reduce gravity ingress of airborne contaminants into the vessels during the drying cycle and subsequent storage between uses.

**Fig. 15** shows the TLWDA of **Fig. 14** in assembled configuration without the lens containers;

**Fig. 16** shows the TLWDA of **Fig. 14** with lens container components oriented for warming or drying;

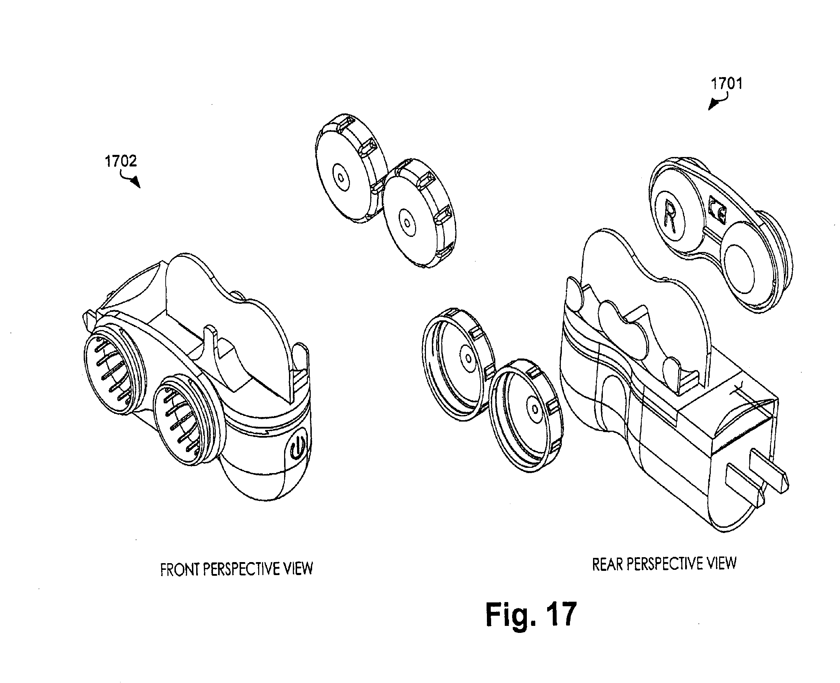

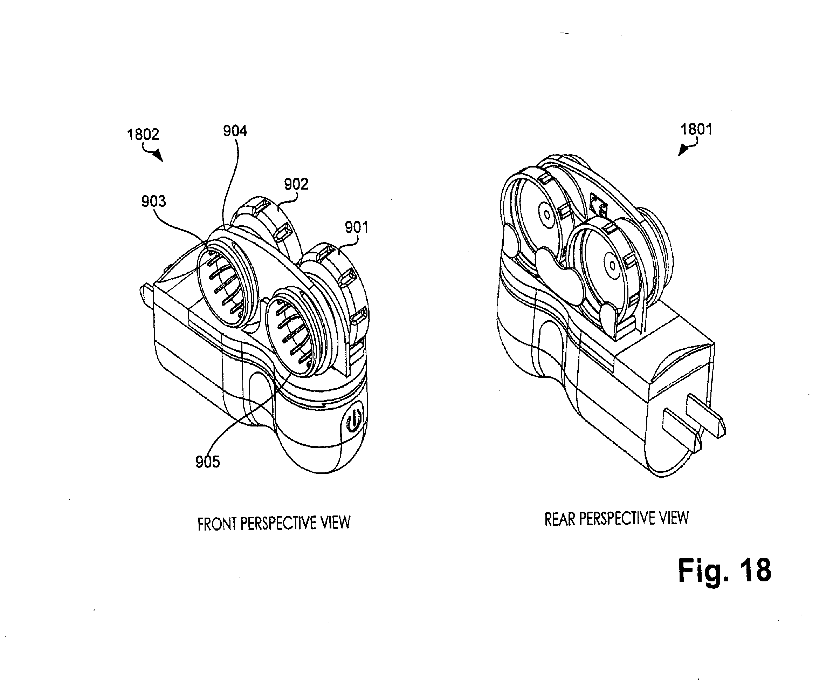

**Fig.** 17 shows a third example of a two-component TLWDA, which does not fall within the scope of the claims *per se,* without lens containers. A rear perspective view shows the heating module 908 engaged with a removable housing 1903. A dovetailed rib (not shown) on the heating module 908 and a complimentary dovetailed channel (not shown) in the removable housing 1903 enable the heating module 908 and the housing 1903 to engage. In this example, the housing 1903 has a vertical plate 1904 on either side of which are drying clips 1905/1906 and 1907. These drying clips are configured to engage with the container lids 901, 902 (see **Fig. 18**) and the vessel/plate assembly 903-905 respectively. Unlike the first and second TLWDA examples shown in **Figs. 12** and **14** respectively, the warming cavities are not accessible when the removable housing 1903 is engaged with the heating module 908. In this example, the housing 1903 is engaged when the drying mode is to be used. When the warming mode is to be used, the housing 1903 is disengaged from the heating module 908, and a removably engageable housing 906 (see **Fig. 9**) can be engaged.

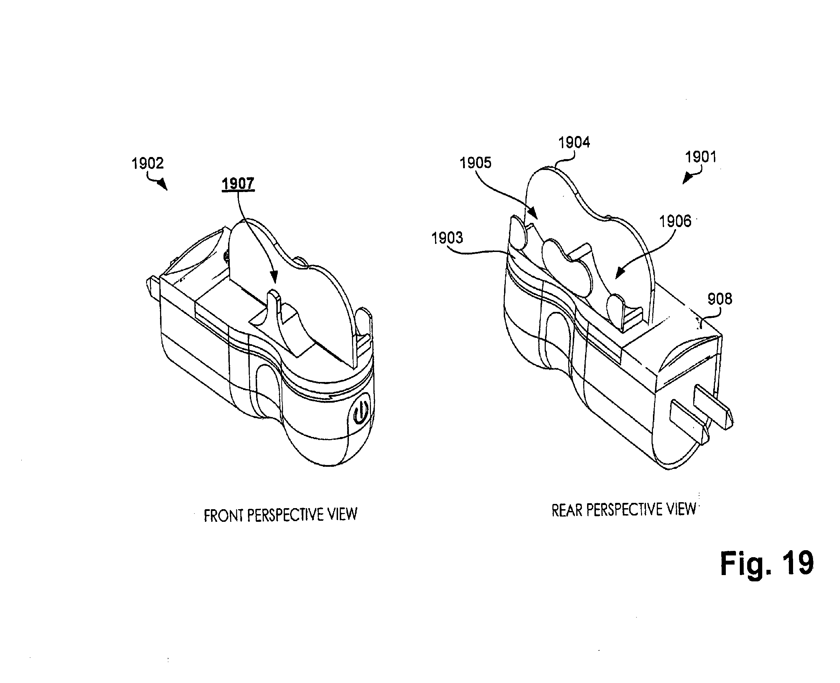

**Fig. 17** shows an exploded mechanical representation of the TLWDA of **Fig. 19** with lens container vessels and lids oriented for drying.

**Fig. 18** shows the TLWDA of **Fig. 17** with lens container vessels and lids mounted in a drying configuration for drying.

A number of technical problems needed to be overcome in the TLWDA. Firstly, it was necessary to minimise the number of device elements in order to reduce the complexity and enable ease of use for the wearer. In the particular expels shown, the TLWDA is a modular combined warmer dryer with interchangeable housings 906, 906/1203/1204 (integrated), and 1402, each having a number of different versions to suit a variety of different container shapes. This enables the wearer to alternate between daily disposable and planned replacement lenses while minimising the number of devices required used. Secondly it was necessary to achieve effective drying while reducing ingress of airborne contaminants. The orientation of the container vessels and lids in the TLWDA arrangements of **Figs. 12****,** **14****,** and **17** achieved this goal. Thirdly it was necessary to achieve effective drying in the drying mode in a sufficiently short time in order to provide the necessary convenience to the wearer and to minimise microbial growth. Thermal pathways for conducting heat to the drying clips in the TLWDA arrangements in **Figs. 12** and **17** were used for this purpose. In the TLWDA arrangement in **Fig. 14****,** convection heating of the lids mounted on the lid supports over the heated container vessels achieved this goal. In general, optimal design of the conformal cavities, time and temperature profiles was also sought in order to meet the requirements.

**Fig. 21** shows a process 2100 of how the TLWDAs of **Figs. 12****,** **14** and **17** can be operated. The process 2100 is described, in one example, by the pseudo-code in APPENDIX B and in a TLWDA arrangement, the process 2100 would be performed, at least in part, by the processor 205 running a software application (not shown) as depicted by the aforementioned pseudo-code. In the example shown the process commences with a step 2101 in which the TLWDA device is in the "Stand-by mode". In a following step 2102 the user removably engages the lens container to the relevant TLWDA device cavity or drying clip. At this point the mating operation depends upon whether the warming mode is to be used or the drying mode is to be used.

Considering the step 2102, if the warming mode is to be used then the lens container such as 902, including fluid, lenses and lids, is engaged with a corresponding heating cavity such as 1306 in the TLWDA arrangement in **Fig. 13****,** or the heating cavity 1503 in the TLWDA arrangement in **Fig, 15****.** The TLWDA arrangement in **Fig. 17** cannot be used as shown for the warming mode, and instead when the warming mode is to be used, the housing 1903 is disengaged from the heating module 908, and a removably engageable housing 906 (see **Fig. 9**) can be engaged. If the warming mode is to be used, then in a subsequent step 2103 the user operates the system operation switch 207 for approximately 1 second. The remainder of the warming operation then follows along the lines of the steps 404 to 407 in **Fig. 3** using temperature and time profiles suitable for warming mode.

Returning to the step 2102, if the drying mode is to be used, then the container vessels and container lids are engaged with the corresponding drying clips 1203, 1204 in **Fig. 13****,** the heating cavities 1503, 1504 and lid drying supports 1603, 1604, in **Figs. 15** and **16** respectively, or the drying clips 1907, 1905/1906 in **Fig. 19****.** If the drying mode is to be used, then in a subsequent step 2103 the user operates the system operation switch 207 for approximately 2 seconds. The remainder of the warming operation then follows along the lines of the steps 404 to 407 in **Fig. 3** using temperature and time profiles suitable for drying mode.

If at any time the switch 207 is operated for approximately 4 seconds, then the TLWDA unit returns to the standby state 2101. The TLWDA unit must be in the standby mode 2101 before it can be operated in either the warming mode or the drying mode.

### Industrial Applicability

It is apparent from the above that the arrangements described are applicable to the domestic appliance and health equipment industries. The foregoing describes only some embodiments of the present invention, and modifications and/or changes can be made thereto without departing from the scope of the invention, the embodiments being illustrative and not restrictive. Accordingly, other temperature ranges and time intervals can be specified, in order to optimise the TLWDA functionality in particular circumstances. These circumstances may depend, among other considerations, upon the prevailing ambient temperature, the age and possibly other demographic variables relating to the users and so on. Other physical arrangements may also be used.

### APPENDIX A - Example Pseudo Code - Warming Mode

```
    Function : pwr_freq_calb()
   Purpose: Determines the frequency of the power supply (currently only chooses
   between 50 and 60 Hz). Sets the variables PWR_OFF_CNST, ERROR_CODE_CNST
   and runs_timer according to the frequency of the power supply so that the behaviour of
   the device is independent of the power supply frequency.
   Input: none
   Returns: none
   Effects: Timing variables
   Called by: The initialisation part of the main procedure
   Synopsis:
   Delay until the start of a power cycle
   Clear the timer register
   Delay until the power cycle has finished
   Delay until the next power cycle starts
   If the timer register is greater than a threshold it is 50 Hz (else 60 Hz)
   Set variables accordingly
  *************************************************************************}
  {*************************************************************************
  Function : delta_check()
  Purpose: Turns the unit off if there is a sudden increase, or if there is absolutely no
  increase in temperature (caused by removing the lens holder, or a broken temp sensor,
  respectively)
  Input: none
  Returns: none
  Effects: previous_temp, can turn unit off
  Called by: Called periodically during the main loop
  Synopsis:
    (Enter this routine periodically, but not on every power cycle)
    Calculate difference (Delta) in temperature, compared to last measurement
    If Temperature is significantly below set point, and Delta is 0,
   Turn Unit off
   If Temperature Delta is too large
   Turn unit off
   Move current temperature into previous temperature variable
   *******************************************************************************}
   {*******************************************************************************
   Function: pwr_on_check()
   Purpose: To check the status of the thyristors before we apply mains power to the
   heating
   elements. Unless both thyristors are activated, no current should pass through the
   heating resistors; hence the test pin should read digital "0".
   Input: none
   Returns: bit (return 1 if there is a problem).
   Effects: Test_var (records problems for debugging - if required)
   Called by: The initialisation part of the main procedure
   Synopsis
  Clear error checking variables
  For each state of the two thyristors (00, 01, 10, 11)
  Wait until not a power cycle (we need the start of a power cycle)
  Wait until a power cycle
  Wait 2 milliseconds (to allow sufficient voltage for testing power components)
  Toggle the low voltage output pins for heaters (if needed) for sufficient time
  Check power circuit
  Record problems in variables (if any)
  Return the value of broken
   (Note: errors are stored in test_var as different bits in a byte, so that multiple problems
   can be
   diagnosed)
   *******************************************************************************}
   {*******************************************************************************
   Function: wait(int)
   Purpose: To allow for an 'accurate' delay of a specified number of milliseconds
   Input: An integer (***current;y signed, probably should be unsigned) between 0 and 127
   Returns: none
   Effects: none
   Called by: various - pwr_on_chk, main
   Synopsis
   Set timer register to use required pre-scaler
   Loop for the number of ms required
   Clear timer
   Wait for timer to reach threshold
   *******************************************************************************}
   {*******************************************************************************
   Function: pulsethyristors ()
   Purpose: To pulse the thyristor "heart-beat monitor" circuit for long enough to allow the
  thyristors time to open and latch. This function operates both thyristors.
   Input: none
  Returns: none
  Effects: Heater output pins
  Called by: Main
  Synopsis
  While not a power cycle
  Pulse Heater outputs (requires a certain time before thyristor will open)
   For a specified period
   Pulse Heater outputs (require a finite time to "latch" the thryistors)
   *******************************************************************************}
   {*******************************************************************************
   Function: heater_state ()
   Purpose: Sets the duty cycle for the heater based on current temperature from the
   sensor.
   Input: None
   Returns: None
  Effects: toohot, zero_cross_count. Reads adresult.
  Called by: Main
  Synopsis:
  Case of adresult
  < Cold then set cold_duty
  < Nearly_Warm set Nearly_Warm Duty
  < Warm set Warm duty
  < Hot set Hot duty
  > Hot turn off and delay for 5 seconds before trying again.
  ******************************************************************************}
  {*******************************************************************************
  Function: ADC_Measure ()
  Purpose: Measures the voltage input on the ADC pin and converts it to a integer.
  Input: None
  Returns: None
  Effects: adres
  Called by: Main
  Synopsis:
  Set ADC read parameters.
    Set AD_Convert flag
    Loop until AD_Result flag set
   Adresult = ADC value.
   *******************************************************************************}
   {*******************************************************************************
   Function: portInit ()
   Purpose: Initialise all the IO ports, Option registers and comparator ports.
   Input: None
   Returns: None
   Effects: TRISB, PORTB, TRISC, PORTC, OPTION, CM2CON0, CM1CON0
   Called by: Main
   Synopsis:
   Set Registers with default values.
   ******************************************************************************}
   {******************************************************************************
   Function: Main ()
   Purpose: Controls the program.
   Input: None
   Returns: None
   Effects: All variables
  Called by: N/A
  Synopsis
  Call Initialisation routines
  Initialise other outputs and variables
  Call frequency calibration check (pwr_freq_calb)
  Call power-on-self-test (pwr_on_chk)
  If power-on-self test fails, and button pushed for 10 seconds
  Display human-readable error code(s)
   [Main loop]
   Turn off heaters, lights and reset running-timer
   If button is pushed
   If button pushed for more than 4 seconds, turn unit off (return to main loop)
   Else (if button pushed for less than 4 seconds) Enter running loop
   [Running Loop]
   If we are in a low power cycle
   If we just finished a positive power cycle
   Increment run timer and turn unit off if set-time is reached (Go to Main Loop)
   Increment duty-counter
  If duty-counter set-value is reached
  Measure temperature (call ADC_Measure)
  Determine which duty cycle to use, and set duty-counter (heater_state)
  If temperature not over-range
  Run a Heat cycle (Call puslethyristors)
  Else leave heaters off.
  Else Wait until next power cycle
  Else go to Running loop
  Else (If we are in a positive power cycle)
  Turn all lights and heaters off go to Running Loop
  *****************************************************************************}
```

### APPENDIX B - Example Pseudo Code - Combined Warming/Drying Modes

```
 Function: pwr_freq_calb()
 Purpose: Determines the frequency of the power supply (currently only chooses between
 50 and 60 Hz). Sets the variables PWR_OFF_CNST, ERROR_CODE_CNST and
 runs_timer according to the frequency of the power supply so that the behaviour of the
 device is independent of the power supply frequency.
 Input: none
 Returns: none
 Effects: Timing variables
 Called by: The initialisation part of the main procedure
 Synopsis:
 Delay until the start of a power cycle
 Clear the timer register
 Delay until the power cycle has finished
 Delay until the next power cycle starts
 If the timer register is greater than a threshold it is 50 Hz (else 60 Hz)
 Set variables accordingly
 *******************************************************************************}
 {*******************************************************************************
 Function : delta_check()
 Purpose: Turns the unit off if there is a sudden increase, or if there is absolutely no
 increase in temperature (caused by removing the lens holder, or a broken temp sensor,
 respectively)
 Input: none
 Returns: none
 Effects: previous_temp, can turn unit off
 Called by: Called periodically during the main loop
 Synopsis:
 (Enter this routine periodically, but not on every power cycle)
 Calculate difference (Delta) in temperature, compared to last measurement
 If Temperature is significantly below set point, and Delta is 0,
 Turn Unit off
 If Temperature Delta is too large
 Turn unit off
 Move current temperature into previous temperature variable
 *******************************************************************************}
 {*******************************************************************************
 Function: pwr_on_check()
 Purpose: To check the status of the thyristors before we apply mains power to the heating
 elements. Unless both thyristors are activated, no current should pass through the heating
 resistors; hence the test pin should read digital "0".
 Input: none
 Returns: bit (return 1 if there is a problem).
 Effects: Test_var (records problems for debugging - if required)
 Called by: The initialisation part of the main procedure
 Synopsis
 Clear error checking variables
 For each state of the two thyristors (00, 01, 10, 11)
 Wait until not a power cycle (we need the start of a power cycle)
 Wait until a power cycle
 Wait 2 milliseconds (to allow sufficient voltage for testing power components)
 Toggle the low voltage output pins for heaters (if needed) for sufficient time
 Check power circuit
 Record problems in variables (if any)
 Return the value of broken
 (Note: errors are stored in test_var as different bits in a byte, so that multiple problems can
 be diagnosed)
 *******************************************************************************}
 {*******************************************************************************
 Function: wait(int)
 Purpose: To allow for an 'accurate' delay of a specified number of milliseconds
 Input: An integer (***current;y signed, probably should be unsigned) between 0 and 127
 Returns: none
 Effects: none
 Called by: various - pwr_on_chk, main
 Synopsis
 Set timer register to use required pre-scaler
 Loop for the number of ms required
 Clear timer
 Wait for timer to reach threshold
 *******************************************************************************}
 {*******************************************************************************
 Function: pulsethyristors ()
 Purpose: To pulse the thyristor "heart-beat monitor" circuit for long enough to allow the
 thyristors time to open and latch. This function operates both thyristors.
 Input: none
 Returns: none
 Called by: Main
 Synopsis
 While not a power cycle
 Pulse Heater outputs (requires a certain time before thyristor will open)
 For a specified period
 Pulse Heater outputs (require a finite time to "latch" the thryistors)
 *******************************************************************************}
 {*******************************************************************************
 Function: heater_state ()
 Purpose: Sets the duty cycle for the heater based on current temperature from the sensor.
 Input: None
 Returns: None
 Effects: toohot, zero_cross_count. Reads adresult.
 Called by: Main
 Synopsis:
 Case of adresult
 < Cold then set cold_duty
 < Nearly_Warm set Nearly_Warm Duty
 < Warm set Warm duty
 < Hot set Hot duty
 > Hot turn off and delay for 5 seconds before trying again.
 *******************************************************************************}
 {*******************************************************************************
 Function: ADC_Measure ()
 Purpose: Measures the voltage input on the ADC pin and converts it to a integer.
 Input: None
 Returns: None
 Effects: adres
 Called by: Main
 Synopsis:
 Set ADC read parameters.
 Set AD_Convert flag
 Loop until AD_Result flag set
 Adresult = ADC value.
 ************************************************************************}
 {************************************************************************
 Function: portInit ()
 Purpose: Initialise all the IO ports, Option registers and comparator ports.
 Input: None
 Returns: None
 Effects: TRISB, PORTB, TRISC, PORTC, OPTION, CM2CON0, CM1CON0
 Called by: Main
 Synopsis:
 Set Registers with default values.
 *******************************************************************************}
 {*******************************************************************************
 Function: Main ()
 Purpose: Controls the program.
 Input: None
 Returns: None
 Effects: All variables
 Called by: N/A
 Synopsis
 Call Initialisation routines
 Initialise other outputs and variables
 Call frequency calibration check (pwr_freq_calb)
 Call power-on-self-test (pwr_on_chk)
 If power-on-self test fails, and button pushed for 10 seconds
 Display human-readable error code(s)
 [Main loop]
 Turn off heaters, lights and reset running-timer
 If button is pushed
 If button pushed for more than 2 seconds, Enter Drying loop
 Else (if button pushed for less than 2 seconds) Enter running loop
 [Running Loop]
 If we are in a low power cycle
 If we just finished a positive power cycle
 Increment run timer and turn unit off if set-time is reached (Go to Main Loop)
 Increment duty-counter
 If duty-counter set-value is reached
 Measure temperature (call ADC_Measure)
 Determine which duty cycle to use, and set duty-counter (heater_state)
 If temperature not over-range
 Run a Heat cycle (Call puslethyristors)
 Else leave heaters off.
 Else Wait until next power cycle
 Else go to Running loop
 Else (If we are in a positive power cycle)
 Turn all lights and heaters off go to Running Loop
 [Drying Loop]
 If we are in a low power cycle
 If we just finished a positive power cycle
 Increment run timer and turn unit off if set-time is reached (Go to Main Loop)
 Increment duty-counter
 If duty-counter set-value is reached
 Measure temperature (call ADC_Measure)
 Determine which duty cycle to use, and set duty-counter (heater_state)
 If temperature not over-range
 Run a Heat cycle (Call puslethyristors)
 Else leave heaters off.
 Else Wait until next power cycle
 Else go to Drying loop
 Else (If we are in a positive power cycle)
 Turn all lights and heaters off go to Drying Loop
 *******************************************************************************}
```

## Claims

1. A contact lens drying apparatus (1401) configured to dry a lens container being removably engaged with the apparatus in a drying configuration, said lens container comprising a container vessel (302, 903, 905) and a lid (301, 901, 902), the lid (902) being dried separately from the container vessel (903), the apparatus comprising:
a controller (106);
drying engagement means (1405, 1406) for removably engaging the container vessel (903) and the lid (901, 902), said drying engagement means comprising:
a lower plate (1406) for engaging the container vessel (903), said lower plate being configured to receive heat from a heating element; and
an upper plate (1405) for engaging the lid (901), said upper plate (1405) being joined to the lower plate (1406) in order to (i) heat the lid and (ii) dispose the lid in an orientation that shields the container vessel to reduce ingress of airborne contaminants; and
the heating element (907); wherein
the controller (106) is configured to direct the heating element (907) to dry the engaged container vessel (903) and the lid (901) thereby significantly reducing microbial activity in the container.

2. A contact lens drying apparatus according to claim 1, wherein:
the heating element is housed in a heating module (908);
the drying engagement means is housed in a housing module (1402) including the drying engagement means (1405, 1406); and
the heating module is configured to mate with said drying engagement means to enable the lens container to be dried.

3. A heating module (908) configured to mate with a drying engagement means (1405, 1406) housed in a housing module (1402), the drying engagement means comprising a lower plate (1406) for engaging a contact lens container vessel (903), said lower plate being configured to receive heat from a heating element; and an upper plate (1405) for engaging a contact lens container lid (901), said upper plate (1405) being joined to the lower plate (1406) in order to (i) heat the lid and (ii) dispose the lid in an orientation that shields the container vessel to reduce ingress of airborne contaminants, said heating module comprising:
a controller (106); and
said heating element (907); wherein
the controller (106) is configured to direct the heating element (907), when the heating module is mated with the drying engagement means (1405, 1406), to dry said container vessel (903) and said lid (901) that are removably engaged with the drying engagement means according to a timing and temperature profile that is adapted for drying, the lid (902) being dried separately from the container vessel (903).

4. A drying engagement means (1405, 1406) housed in a housing module (1402), wherein the drying engagement means is configured to mate with a heating module (908) which, when mated with the drying engagement means, is configured to dry a lens container comprising a container vessel and a lid that is removably engaged with the drying engagement means, the lid (902) being dried separately from the container vessel (903), said drying engagement means comprising:
a lower plate (1406) for engaging the container vessel (903), said lower plate being configured to receive heat from the engaged heating module (908),
an upper plate (1405) for engaging the lid (902), said upper plate being joined to the lower plate in order to (i) heat the lid and (ii) dispose the lid in an orientation that shields the container vessel to reduce ingress of airborne contaminants; and
means (1404) for mating with the heating module.

## Patentansprüche

1. Kontaktlinsentrocknungsvorrichtung (1401), die konfiguriert ist, um einen Linsenbehälter zu trocknen, der mit der Vorrichtung in einer Trocknungskonfiguration entfernbar in Eingriff ist, wobei der Linsenbehälter ein Behältergefäß (302, 903, 905) und einen Deckel (301, 901, 902) beinhaltet, wobei der Deckel (902) getrennt vom Behältergefäß (903) getrocknet wird, wobei die Vorrichtung Folgendes beinhaltet:
eine Steuereinheit (106);
Trocknungseingreifmittel (1405, 1406), um das Behältergefäß (903) und den Deckel (901, 902) entfernbar in Eingriff zu bringen, wobei das Trocknungseingreifmittel Folgendes beinhaltet:
eine untere Platte (1406), um das Behältergefäß (903) in Eingriff zu bringen, wobei die untere Platte konfiguriert ist, um von einem Heizelement Wärme zu empfangen; und
eine obere Platte (1405), um den Deckel (901) in Eingriff zu bringen, wobei die obere Platte (1405) an die untere Platte (1406) angefügt ist, um (i) den Deckel zu erwärmen und (ii) den Deckel in einer Orientierung anzuordnen, die das Behältergefäß abschirmt, um den Eintritt von luftgetragenen Verunreinigungen zu reduzieren; und
das Heizelement (907); wobei
die Steuereinheit (106) konfiguriert ist, um das Heizelement (907) anzuweisen, das in Eingriff befindliche Behältergefäß (903) und den Deckel (901) zu trocknen, wodurch die mikrobielle Aktivität im Behälter signifikant reduziert wird.

2. Kontaktlinsentrocknungsvorrichtung gemäß Anspruch 1, wobei:
das Heizelement in einem Heizmodul (908) untergebracht ist;
das Trocknungseingreifmittel in einem Gehäusemodul (1402) untergebracht ist, das das Trocknungseingreifmittel (1405, 1406) einschließt; und
das Heizmodul konfiguriert ist, um mit dem Trocknungseingreifmittel zusammengepasst zu werden, um zu ermöglichen, dass der Linsenbehälter getrocknet wird.

3. Heizmodul (908), das konfiguriert ist, um mit dem Trocknungseingreifmittel (1405, 1406) zusammengepasst zu werden, das in einem Gehäusemodul (1402) untergebracht ist, wobei das Trocknungseingreifmittel eine untere Platte (1406) beinhaltet, um ein Kontaktlinsenbehältergefäß (903) in Eingriff zu bringen, wobei die untere Platte konfiguriert ist, um von einem Heizelement Wärme zu empfangen; und eine obere Platte (1405)beinhaltet, um einen Kontaktlinsenbehälterdeckel (901) in Eingriff zu bringen, wobei die obere Platte (1405) an die untere Platte (1406) angefügt ist, um (i) den Deckel zu erwärmen und (ii) den Deckel in einer Orientierung anzuordnen, die das Behältergefäß abschirmt, um den Eintritt von luftgetragenen Verunreinigungen zu reduzieren, wobei das Heizmodul Folgendes beinhaltet:
eine Steuereinheit (106); und
das Heizelement (907); wobei
die Steuereinheit (106) konfiguriert ist, um das Heizelement (907) anzuweisen, wenn das Heizmodul mit dem Trocknungseingreifmittel (1405, 1406) zusammengepasst ist, das Behältergefäß (903) und den Deckel (901) zu trocknen, die mit dem Trocknungseingreifmittel entfernbar in Eingriff sind, gemäß einem Zeitsteuerungs- und Temperaturprofil, das zum Trocknen angepasst ist, wobei der Deckel (902) getrennt vom Behältergefäß (903) getrocknet wird.

4. Trocknungseingreifmittel (1405, 1406), das in einem Gehäusemodul (1402) untergebracht ist, wobei das Trocknungseingreifmittel konfiguriert ist, um mit einem Heizmodul (908) zusammengepasst zu werden, das, wenn mit dem Trocknungseingreifmittel zusammengepasst, konfiguriert ist, um einen Linsenbehälter zu trocknen, der ein Behältergefäß und einen Deckel beinhaltet, und der mit dem Trocknungseingreifmittel entfernbar in Eingriff ist, wobei der Deckel (902) getrennt vom Behältergefäß (903) getrocknet wird, wobei das Trocknungseingreifmittel Folgendes beinhaltet:
eine untere Platte (1406), um das Behältergefäß (903) in Eingriff zu bringen, wobei die untere Platte konfiguriert ist, um von dem in Eingriff befindlichen Heizmodul (908) Wärme zu empfangen;
eine obere Platte (1405), um den Deckel (902) in Eingriff zu bringen, wobei die obere Platte an die untere Platte angefügt ist, um (i) den Deckel zu erwärmen und (ii) den Deckel in einer Orientierung anzuordnen, die das Behältergefäß abschirmt, um den Eintritt von luftgetragenen Verunreinigungen zu reduzieren; und
Mittel (1404) zum Zusammenpassen mit dem Heizmodul.

## Revendications

1. Appareil de séchage de lentilles de contact (1401) configuré pour sécher un contenant pour lentilles qui est relié de manière amovible à l'appareil dans une configuration de séchage, ledit contenant pour lentilles comprenant un récipient (302, 903, 905) et un couvercle (301, 901, 902), le couvercle (902) étant séché séparément du récipient (903), l'appareil comprenant :
un contrôleur (106) ;
un moyen de liaison pour le séchage (1405, 1406) qui sert à relier le récipient (903) de manière amovible au couvercle (901, 902), ledit moyen de liaison pour le séchage comprenant :
une plaque inférieure (1406) destinée à être reliée au récipient (903), ladite plaque inférieure étant configurée pour recevoir de la chaleur fournie par un élément chauffant ; et
une plaque supérieure (1405) destinée à être reliée au couvercle (901), ladite plaque supérieure (1405) étant assemblée avec la plaque inférieure (1406) de façon à (i) chauffer le couvercle et (ii) disposer le couvercle dans une orientation qui protège le récipient de manière à réduire la pénétration de contaminants en suspension dans l'air ; et
l'élément chauffant (907) ; dans lequel
le contrôleur (106) est configuré pour commander l'élément chauffant (907) afin qu'il sèche le récipient (903) et le couvercle (901) reliés, ce qui a pour effet de réduire significativement l'activité microbienne dans le contenant.

2. Appareil de séchage de lentilles de contact selon la revendication 1, dans lequel :
l'élément chauffant est logé dans un module de chauffage (908) ;
le moyen de liaison pour le séchage est logé dans un module de logement (1402) qui contient le moyen de liaison pour le séchage (1405, 1406) ; et
le module de chauffage est configuré pour être accouplé avec ledit moyen de liaison pour le séchage afin de permettre le séchage du contenant pour lentilles.

3. Module de chauffage (908) configuré pour être accouplé avec un moyen de liaison pour le séchage (1405, 1406) logé dans un module de logement (1402), le moyen de liaison pour le séchage comprenant une plaque inférieure (1406) destinée à être reliée à un récipient pour lentilles de contact (903), ladite plaque inférieure étant configurée pour recevoir de la chaleur fournie par un élément chauffant ; et une plaque supérieure (1405) destinée à être reliée à un couvercle de contenant pour lentilles de contact (901), ladite plaque supérieure (1405) étant assemblée avec la plaque inférieure (1406) de façon à (i) chauffer le couvercle et (ii) disposer le couvercle dans une orientation qui protège le récipient de manière à réduire la pénétration de contaminants en suspension dans l'air, ledit module de chauffage comprenant :
un contrôleur (106) ; et
ledit élément chauffant (907) ; dans lequel
le contrôleur (106) est configuré pour commander l'élément chauffant (907), lorsque le module de chauffage est accouplé avec le moyen de liaison pour le séchage (1405, 1406), afin qu'il chauffe ledit récipient (903) et ledit couvercle (901) qui sont reliés de manière amovible au moyen de liaison pour le séchage selon un profil de temps et de température qui est adapté en vue du séchage, le couvercle (902) étant séché séparément du récipient (903).

4. Moyen de liaison pour le séchage (1405, 1406) logé dans un module de logement (1402), dans lequel le moyen de liaison pour le séchage est configuré pour être accouplé avec un module de chauffage (908) qui, lorsqu'il est accouplé avec le moyen de liaison pour le séchage, est configuré pour sécher un contenant pour lentilles qui comprend un récipient et un couvercle et qui est relié de manière amovible au moyen de liaison pour le séchage, le couvercle (902) étant séché séparément du récipient (903), ledit moyen de liaison pour le séchage comprenant :
une plaque inférieure (1406) destinée à être reliée au récipient (903), ladite plaque inférieure étant configurée pour recevoir de la chaleur fournie par le module de chauffage (908) relié ;
une plaque supérieure (1405) destinée à être reliée au couvercle (902), ladite plaque supérieure étant assemblée avec la plaque inférieure de façon à (i) chauffer le couvercle et (ii) disposer le couvercle dans une orientation qui protège le récipient de manière à réduire la pénétration de contaminants en suspension dans l'air ; et
un moyen (1404) d'accouplement avec le module de chauffage.
